(19) Europäisches Patentamt
European Patent Office
Office européen des brevets

(11) Publication number: 0 014 437
B1

(12) **EUROPEAN PATENT SPECIFICATION**

(45) Date of publication of patent specification: 23.02.83

(21) Application number: 80100477.1

(22) Date of filing: 31.01.80

(51) Int. Cl.[3]: C 07 C 103/38, C 07 C 147/12, C 07 C 147/14, C 07 C 149/42, C 07 C 143/80, C 07 C 147/107, C 07 C 117/08, C 07 C 91/18, C 07 D 209/48, A 61 K 31/16, C 07 B 23/00

(54) **Novel 1-aryl-2-acylamido-3-fluoro-1-propanols, their preparation and pharmaceutical compositions containing them.**

(30) Priority: 05.02.79 US 9207

(43) Date of publication of application: 20.08.80 Bulletin 80/17

(45) Publication of the grant of the patent: 23.02.83 Bulletin 83/8

(84) Designated Contracting States: AT BE CH DE FR GB IT NL SE

(56) References cited: GB - A - 1 091 448

(73) Proprietor: SCHERING CORPORATION
2000 Galloping Hill Road
Kenilworth, New Jersey 07033 (US)

(72) Inventor: Nagabhushan, Tattanahalli, Lakshminarayan
3 Sunset Lane
Parsippany Morris, New Jersey 07054 (US)

(74) Representative: Antony, Fritz, Dr. et al,
P.O. Box 601 Winkelriedstrasse 35
CH-6002 Lucerne (CH)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 014 437 B1

# Novel 1-aryl-2-acylamido-3-fluoro-1-propanols, their preparation and pharmaceutical compositions containing them

This invention relates to novel 1-aryl-2-acylamido-3-fluoro-1-propanols, to their preparation and to pharmaceutical compositions containing the same.

Known in the art are certain broad spectrum antibiotics which may be classified as D-(*threo*)-1-*p*-substituted phenyl-2-dihaloacetamido-1,3-propanediols, this class of antibiotics being exemplified by chloramphenicol (D-(*threo*)-1-*p*-nitrophenyl-2-dichloroacetamido-1,3-propanediol), thiamphenicol (D-(*threo*)-1-*p*-methylsulfonylphenyl-2-dichloroacetamido-1,3-propanediol), fluorthiamphenicol (D-(*threo*)-1-*p*-methylsulfonylphenyl-2-difluoroacetamido-1,3-propanediol), tevenel (D-(*threo*)-1-*p*-aminosulfonylphenyl-2-dichloroacetamido-1,3-propanediol) and fluortevenel which is the difluoroacetamido analog of tevenel. Also known are various esters of the foregoing of which the palmitate and hemisuccinate may be mentioned as examples.

It is known that, in general, structural modifications may be made in the 2-haloacetamido-side chain and in the phenyl moiety of the basic chloramphenicol molecule of the foregoing class without significant loss of biological activity occurring. However, structural modifications at the primary hydroxy group at position 3 of the propanediol moiety, including replacement by chlorine or bromine, have hitherto been shown to destroy or significantly reduce biological activity. See for instance:
F. E. Hahn, *Antibiotics*, Ed. Gottlieb and Shaw, Springer-Verlag, New York, (1967), p. 308,
F. E. Hann et al, *Antibiotics and Chemotherapy, 6,* No. 9, 531 (1956),
L. Cima and A. Ilecto, *Il Farmaco,* Ed. Sc. *12,* No. 6, 535 (1957),
S. Mitsuhasi et al, *Jap. J. Microbiol. 13,* No. 2, 177—80 (1969), and
M. Kono et al, *Jap. J. Microbiology 15* (3), 219—27 (1971).

We have now surprisingly found that the 3-hydroxy group of the propanediol moiety of the basic chloramphenicol molecule may be replaced by fluorine without destroying the bioligical activity of the molecule. Indeed in this manner novel 3-fluoro-derivatives are obtainable which are broad spectrum antibacterial agents useful in the treatment of gram-positive, gram-negative and rickettsial infections and which may also be cidal against bacterial resistant to chloramphenicol and related known antibiotics of this group.

Accordingly, in one of its aspects the present invention provides novel D-(*threo*)-1-aryl-2-acylamido-3-fluoro-1-propanol compounds of the general formula I

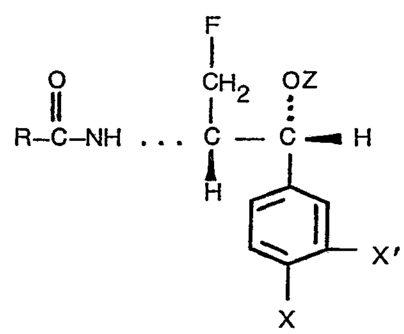

(I),

in which formula:
R represents an alkyl group having one or two carbon atoms which group may be substituted by one or more halogen atoms, or represents azidomethyl, methylsulfonylmethyl or a halodeuteroalkyl group

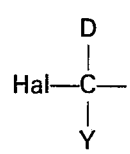

where Y is $—CH_3$, $—CH_2Hal$ or Hal, and Hal represents a halogen atom,
X and X′ independently represents a hydrogen or halogen atom, a nitro, cyano or phenyl group which phenyl group may be substituted by halogen or by lower alkyl, lower alkoxy, methylsulfonyl or nitro, or represent a group $R_1(A)_n$ in which group n is zero or 1, A is oxygen, sulfur, carbonyl, sulfinyl or sulfonyl and $R_1$ is lower alkyl, or when A is sulfinyl or sulfonyl then $R_1$ may also be amino or lower alkyl amino, the aforementioned lower alkyl or lower alkoxy groupings having one to three carbon atoms, and
Z is hydrogen or an acyl radical of a carboxylic acid having up to 16 carbon atoms.

Also included within the scope of the present invention are the pharmaceutically acceptable salts of the compounds of the formula I where Z is an acyl radical derived from a dicarboxylic acid or amino acid having up to 16 carbon atoms.

The group R may be methyl, ethyl, azidomethyl, methylsulfonylmethyl or more preferably a methyl

or ethyl group substituted by one or more halogen atoms or the aforementioned group

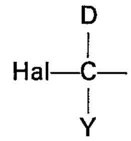

where Y and Hal are as defined for formula I.

Of the halo-substituted alkyl groups, the preferred groups are halo-substituted methyl groups with the halo-substituent being preferably a fluoro or chloro substituent. Specifically, the grouping $(Hal)_2.CH$— may be mentioned where Hal, which may be the same or different, represents a fluorine or chlorine atom, i.e. $F_2CH$—, $CL_2CH$— and FClCH—. Other examples of halo-substituted methyl groups are mono- and trifluoromethyl, mono- and trichloromethyl, mono- and dibromomethyl, and mono- and diiodomethyl. As examples of mixed halo-substituted methyl groups there may be mentioned bromochloromethyl, bromofluoromethyl, dichlorofluoromethyl and difluorochloromethyl.

The preferred halo-substituted ethyl groups are $\alpha$- and $\beta$-monohalo-substituted ethyl and $\alpha,\alpha$- and $\alpha,\beta$-dihalo-substituted ethyl groups. Again, in the polyhalo-substituted groups the halo-substituents may be the same or different. The halo-substituent is preferably fluoro or chloro.

The deuteroalkyl group

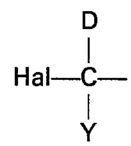

included within the definition of R, where Y and Hal are as defined for formula I, encompasses the groups

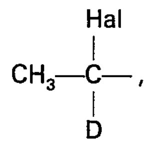

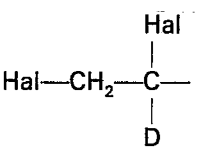

and $(Hal)_2CD$— of which $(Hal)_2CD$— is preferred. The halo-substituents which may be the same or different are preferably fluoro or chloro.

In the definitions of X and X' in formula I, the group $R_1(A)_n$ specifically includes —$SO_2R_1$, —$SOR_1$, —$SR_1$, —$SO_2NH_2$, —$SONH_2$, —$SO_2NHR_1$, —$SONHR_1$, —$COR_1$, —$OR_1$ and $R_1$ where $R_1$ is as defined for formula I being isopropyl, *n*-propyl, ethyl or preferably methyl.

While the precise nature of X and X' is not considered critical for the purposes of the present invention, a preferred area of compounds are those where X' is hydrogen and X is —$SO_2NH_2$, —$SOCH_3$ and in particular —$SO_2CH_3$ and nitro.

The two asymmetric centers in the formula I compounds give rise to four stereoisomers of which, as shown, the compounds of the present invention have the absolute configuration D and the relative configuration *threo*. That is, the compounds of the present invention have the same configuration as the prior art antibiotics chloramphenicol, its difluoroacetyl analog thiamphenicol, fluorothiamphenicol and tevenel.

For the compounds of the formula I, Z is hydrogen or, as stated, an acyl radical of a carboxylic acid having up to 16 carbon atoms. The acyl radicals of the carboxylic acids having up to 16 carbon atoms may be saturated or unsaturated, straight-chain or branched-chain, aliphatic, alicyclic, alicyclic-aliphatic, aromatic, aryl-aliphatic, alkyl-aromatic or heterocyclic, and include such radicals derived from amino acids and hydroxy acids, and, further, may be substituted by one or more halogen atoms or by alkoxy groups containing 1 to 5 carbon atoms.

Typical acyl radicals are those derived from hydrocarbon-carboxylic acids such as alkanoic acids exemplified by the aliphatic acids formic, acetic, propionic, butyric, isobutyric, valeric, isovaleric, pivalic, hexanoic, t-butylacetic, heptanoic, octanoic, decanoic, undecylic, lauric and plamitic; substituted

aliphatic acids e.g. phenoxyacetic, trifluoroacetic, β-chloropropionic and pantothenic acids; alicyclic and alicyclic-aliphatic acids such as cyclopentylpropionic and adamantanecarboxylic acids; aromatic and substituted aromatic acids such as benzoic, toluic, *p*-chlorobenzoic, *p*-fluorobenzoic, *p*-methoxybenzoic and 3′,5′-dimethylbenzoic acids; aryl aliphatic acids such as phenylacetic, phenylpropionic and β-benzoylamino-isobutyric acids; unsaturated acids such as acrylic, cinnamic and sorbic acids and, preferably, dibasic acids such as phthalic acid and succinic acid and dibasic hydroxy acids such as tartaric acid.

The preferred amino acids from which the acyl radical representing Z may be derived are amino acids containing up to 12 carbon atoms as exemplified by tryptophan, threonine, serine, hydroxyproline, proline, tyrosine, phenylalanine, isoleucine, valine, alanine, glycine, ornithine, lysine and diaminobutyric acid.

Preferred esters of the general formula I are those capable of forming pharmaceutically acceptable salts, in particular water-soluble pharmaceutically acceptable salts, such esters being those where Z in the general formula I is an acyl group derived from a dicarboxylic acid or an amino acid.

For acid esters, that is where Z is a monovalent acyl radical derived from a dicarboxylic acid such as succinic acid, suitable salts are cationic salts such as alkali-metal or alkaline-earth metal salts, e.g. sodium, potassium and calcium salts and also aluminium salts. Other suitable cationic salts are those formed with amines such as trialkylamines, procaine, dibenzylamine, *N*-benzyl-betaphenethylamine, *N,N′*-dibenzylethylenediamine, *N,N′*-bis-dehydroabietylethylenediamine, *N*-(lower)alkylpiperidines (e.g. N-ethylpiperidine), and *N*-methyl-glucamine.

These cationic salts may be prepared in known manner such as by treating the acid esters, for instance the hemisuccinate, in aqueous solution with an equimolar amount of the required base, for instance sodium hydroxide or trimethylamine, and lyophilizing the resulting solution.

Where Z is an acyl radical derived from an amino acid, suitable salts are acid addition salts formed with mineral or organic acids such as hydrochloric, sulfuric, phosphoric, nitric, hydrobromic, acetic, propionic, mallic, ascorbic, citric or succinic acids. Preferably the amino acid from which Z is derived is an α-amino acid such as glycine. Preferred ester acid addition salts are the glycinate hydrochloride and sulfate salt. The acid addition salts may be prepared in known manner by neutralising the basic amino acid ester, for instance the glycinate ester, with the appropriate amount of the required acid, for instance hydrochloric, to give a resulting pH of about 4.5 to 5.

Preferred compounds of the invention include:

D-(*threo*)-1-*p*-nitrophenyl-2-dichloroacetamido-3-fluoro-1-propanol,

D-(*threo*)-1-*p*-nitrophenyl-2-difluoroacetamido-3-fluoro-1-propanol,

D-(*threo*)-1-*p*-methylsulfonylphenyl-2-dichloroacetamido-3-fluoro-1-propanol,

D-(*threo*)-1-*p*-methylsulfonylphenyl-2-difluoroacetamido-3-fluoro-1-propanol,

D-(*threo*)-1-*p*-nitrophenyl-2-(R,S-chlorofluoroacetamido)-3-fluoro-1-propanol and

D-(*threo*)-1-*p*-methylsulfonylphenyl-2-(R,S-chlorofluoroacetamido)-3-fluoro-1-propanol,

as well as the corresponding 2-dihalodeuterio-analogs of the foregoing such as:

D-(*threo*)-1-*p*-nitrophenyl-2-dichlorodeuterioacetamido-3-fluoro-1-propanol,

D-(*threo*)-1-*p*-nitrophenyl-2-difluorodeuterioacetamido-3-fluoro-1-propanol,

D-(*threo*)-1-*p*-methylsulfonylphenyl-2-dichlorodeuterioacetamido-3-fluoro-1-propanol, and

D-(*threo*)-1-*p*-methylsulfonylphenyl-2-difluorodeuterioacetamido-3-fluoro-1-propanol.

Other compounds which may be specifically mentioned are:

D-(*threo*)-1-*p*-aminosulfonylphenyl-2-dichloroacetamido-3-fluoro-1-propanol,

D-(*threo*)-1-*p*-aminosulfonylphenyl-2-difluoroacetamido-3-fluoro-1-propanol,

D-(*threo*)-1-*p*-methylsulfinylphenyl-2-dichloroacetamido-3-fluoro-1-propanol, and

D-(*threo*)-1-*p*-methylsulfinylphenyl-2-difluoroacetamido-3-fluoro-1-propanol.

Preferred ester derivatives of the compounds of the invention are the hemisuccinate esters particularly in the form of the sodium and trimethylamine cationic salts, and the glycinate ester particularly in the form of its hydrochloride or sulfate acid addition salt. As specifically preferred ester derivatives there may be mentioned the sodium and trimethylamine salts of D-(*threo*)-1-*p*-nitrophenyl-2-dichloroacetamido-3-fluoro-1-propyl hemisuccinate and the hydrochloride and sulfate acid addition salts of D-(*threo*)-1-*p*-nitrophenyl-2-dichloroacetamido-3-fluoro-1-propyl glycinate. The pharmaceutically acceptable salts of the specified compounds of the general formula I, such as the aforementioned salts, are usually white or off-white solids which are generally soluble in water, sparingly soluble in polar solvents and insoluble in non-polar solvents.

In another of its aspects the present invention provides methods for the preparation of the compounds of the general formula I.

A first general method for the preparation of the compounds of the general formula I comprises N-acylating an amine of the general formula II

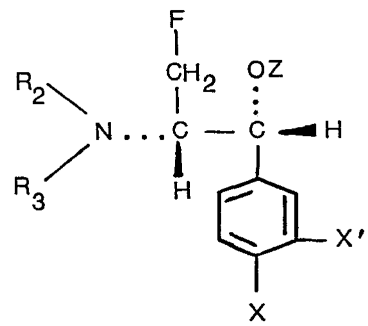

(II),

wherein X, X' and Z are as defined in formula I, Z being most preferably hydrogen, and $R_2$ and $R_3$ are each hydrogen, with a free acid or with a reactive derivative of an acid of the formula R.COOH where R is as defined in claim 1.

The N-acylation may be effected in manner known per se, the reactive derivative of the acid R.COOH suitably being an acid anhydride, an acid halide (for example the acid chloride) or, particularly where R is a halo-substituted methyl or ethyl group, a lower alkyl ester derivative (for example a methyl ester).

The 2-unsubstituted amino-3-fluoro-1-propanol compounds of the general formula II, as well as the corresponding N-protected-amino compounds where $R_2$ and $R_3$ individually or together represent protecting groups, are novel compounds which may be prepared by a process which includes the step of replacing with a fluorine atom the hydroxy group at the 3-position of a corresponding 2-N-protected amino-1,3-propanediol compound of the general formula III,

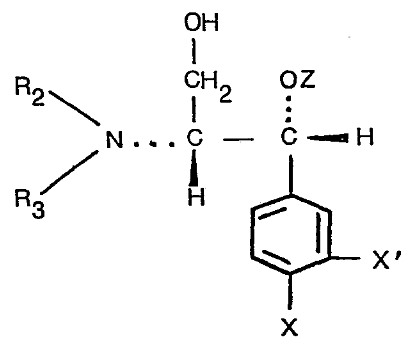

(III),

whereby the 3-primary hydroxy function is replaced by fluorine, wherein in formula III, X, X' and Z are as defined for formula I, Z being most preferably hydrogen, and $R_2$ and $R_3$ individually or together represent amino protecting groups. While any suitable protecting groups may be used, preferred groups are those derived from dicarboxylic acids such as phthalic acid and succinic acid where the grouping $R_2R_3N$— then represents phthalimido and succinimido respectively. The free amino compounds corresponding to the formula III compounds are known compounds which may be protected at the amino function in conventional manner.

The fluorination step may be effected using as fluorinating agent a sulfurtrifluoride-secondary amine adduct such as sulfurtrifluoride-morpholine, sulfurtrifluoridepiperidine or, preferably, a dialkylamine-sufurtrifluoride such as diethylamine-sulfurtrifluoride.

Typically, fluorination may be effected using equimolar quantities of the sulfur trifluoride-secondary amine adduct and the starting material of the general formula III in an inert solvent and at a temperature lying within the range of from about −10° to +50°C, preferably from about 0°C up to room temperature. The resulting compound corresponding to that of the general formula II except that $R_2$ and $R_3$ represent N-amino protecting groups may then be treated to remove these protecting groups utilizing known techniques to give the required free amino compound of the general formula II. Where for instance the grouping $R_2R_3N$— is phthalimido, then the corresponding protecting group may suitably be removed by treatment in ethanol with hydrazine hydrate or hydroxylamine.

A further general method for the preparation of the compounds of the general formula I comprises ring opening, with concomitant fluorination at the 3-position of the propane moiety, of an oxazoline of the general formula IV

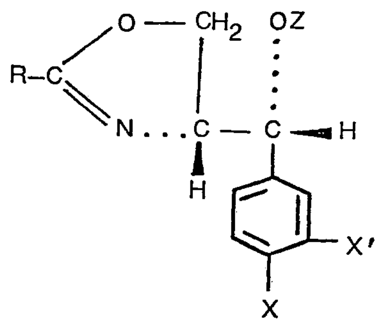

(IV)

wherein R, X, X′ and Z are as defined for formula I, Z being most preferably hydrogen. The ring opening may suitably be achieved by treating the oxazoline with hydrogen fluoride in the presence of a fluoride ion for example lithium fluoride and preferably in the presence of an acid catalyst. especially boron trifluoride dietherate. The reaction may be carried out using liquid hydrogen fluoride as the reaction medium taking suitable precautions such as using a teflon-lined bomb as the reaction vessel.

The oxazoline starting materials may be prepared in known manner. Thus a starting material corresponding to that of the required end product of the general formula I except for having a 3-hydroxy group in place of the 3-fluoro substituent (for instance chloramphenicaol itself) may be converted into the corresponding trifluoromethylsulfonate or 2-*p*-toluene-sulfonate ester which is then cyclised upon treatment with a base. Conversion to the required sulfonate ester may conveniently be effected using trifluoromethylsulfonyl chloride in pyridine or *p*-toluenesulfonyl chloride in pyridine.

Another general method for preparing the compounds of the general formula I comprises oxidising a starting material differing from the desired product in having in one or both of the para- and meta-positions of the phenyl moiety a substituent $—SR_1$ or $—SOR_1$, where $R_1$ is as defined for formula I, to effect conversion of said substituent into $—SOR_1$ and $—SO_2R_1$ respectively.

This method is applicable for instance to the preparation of compounds of the general formula I where X′ is hydrogen and X is $—SOR_1$ or $—SO_2R_1$. Such compounds may be obtained by oxidation of a corresponding starting material in which X′ is hydrogen and X″ is respectively $—SR_1$ and $—SOR_1$ using oxidising agents and conditions commonly employed in the conversion of an aryl thio-ether group into sulfinyl and for the conversion of an aryl sulfinyl group into sulfonyl. For example in the conversion of thioether to sulfinyl we have employed sodium metaperiodate as oxidising agent.

A further method for the preparation of those compounds of the general formula I where R is

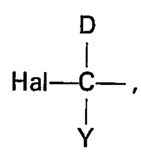

Y being as defined for formula I, comprises deuterating a corresponding starting material of the general formula I where R is

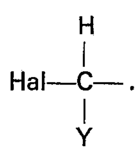

Deuteration may be effected using conventional deuterium exchange reactions, for instance we have employed methyl alcohol-d (that is $CH_3OD$).

Various optional finishing steps may be applied to the compounds obtained by the foregoing processes, for instance esterification or hydrolysis at the 1-hydroxy function and formation of a pharmaceutically acceptable salt. These finishing steps may be carried out using known techniques.

Compounds of the general formula I where Z is an acyl radical are preferably prepared by appropriate esterification of the corresponding compound of the general formula I in which Z is hydrogen, the so-obtained ester being isolated as such or where appropriate in the form of a pharmaceutic-ally acceptable salt.

The preparation of the compounds of the general formula I will now be illustrated by means of the following Examples.

Example 1
D-(*THREO*)-1-*p*-NITROPHENYL-2-DICHLOROACETAMIDO-3-FLUORO-1-PROPANOL

A. D-(Threo)-1-*p*-Nitrophenyl-2-Phthalimido-1,3-Propanediolropanol,

Add D-(*threo*)-1-*p*-nitrophenyl-2-amino1,3-propanediol (21.2 gm., 0.1 mole), triethylamine (15 ml.) and phthalic anhydride (14.8 gm., 0.1 mole) to toluene (600 ml.) in a flask equipped with an overhead stirrer and a Dean and Stark water collector. Stir the reaction mixture at reflux temperature for 4 hours, cool with stirring to about 50°C., then add ethanol (300 ml.). Stir at room temperature for 1 hour, then allow the reaction mixture to remain at room temperature for 18 hours. Separate the resultant white precipitate by filtration, wash the residue with ethanol and dry to obtaind D-(*threo*)-1-*p*-nitrophenyl-2-phthalimido-1,3-propanediol, yeild 28.4 gm. (83% theory); m.p. 228—229°C (decomp.). Mass Spectrum: ($M^+$+1)343.PMR(dmso-$d_6$): $\delta$ 6.93—8.33 (aromatic hydrogens), 5.93 (d, J=4.0 Hz, benzylic OH), 5.27 (m,H—1), 4.83 (broad t, primary OH), 3.77—4.62 (m, $CH_2$), 3.5 (m, H—2).

B. D-(*Threo*)-1-*p*-Nitrophenyl-2-Phthalimido-3-Fluoro-1-propanol

To a stireed solution of D-(threo)-1-*p*-nitrophenyl-2-phthalimido-1,3-propanediol (42.76 gm., 0.125 mole) in dry tetrahydrofuran (800 ml.) maintained at 0°C., add dropwise diethylaminosulfurtrifluoride (17.5 ml.). Stir the reaction mixture at 0°C. for 30 minutes, then allow the reaction mixture to warm to room temperature and stir at room temperature for an additional 5 hours. Evaporate the tetrahydrofuran *in vacuo* and chromatograph the resultant residue on a column of silica gel (2 kg.) eluting with a mixture of chloroform and ethanol (99:1 by volume). Combine the homogenous eluates containing the desired product as determined by thin layer chromatography and evaporate *in vacuo* to give a residue of D-(*threo*)-1-*p*-nitrophenyl-2-phthalimido-3-fluoro-1-propanol, yield 32 gm. (74.4% theory). Further purify by crystallization from ethyl acetate:petroleum ether; m.p. 188—190°C., $[\alpha]_D^{26}$ = −55.6° (1% in dimethylformamide). Mass Spectrum: ($M^+$+1) 345, $M^+$ 344. PMR dmso-$d_6$ $\delta$ 7.70—8.42 (aromatic hydrogens). 6.20 (d, J=4.0 Hz, benzylic OH), 5.3 (m H—1), 4.1—4.95 (m, $CH_2F$, H—2). Analysis: Calculated for $C_{17}H_{13}N_2O_5F$: C=59.29%, H=3.80%, N=8.13%, F=5.52%; Found: C=59.17%, H=3.93%, N=7.80%, F=5.40%.

C. D-(*Threo*)-1-*p*-Nitrophenyl-2-Amino-3-Fiuoro -1-Propanol

Heat a mixture of D-(*threo*)-1-*p*-nitrophenyl-2-phthalimido-3-fluoro-1-propanol (25.8 gm., 5 mmol), hydrazine hydrate (99%, 4 gm., 80 mmol), and ethanol (460 ml.) at reflux temperature for 4 hours, then let the reaction mixture stand at room temperature for 18 hours. Separate the solids by filtration and wash with a little ethanol. Concentrate the combined filtrate and ethanol washings *in vacuo* and extract the resultant residue with a chloroform/ethanol mixture (90:10 by volume) (700 ml.). Combine the chloroform/ethanol extracts and evaporate *in vacuo* to give 18.1 gm. of a residue comprising D-(*threo*)-1-*p*-nitrophenyl-2-amino-3-fluoro-1-propanol, which can be used without further purification in the procedure described in the following example.

To prepare an analytical sample, chromatograph the foregoing residue in silica gel eluting with a mixture of chloroform, methanol and ammonium hydroxyde (30:5:0.1 by volume). Combine the like fractions containing the desired product as determined by thin layer chromatography and evaporate the combined eluates to a residue comprising D-(*threo*)-1-*p*-nitrophenyl-2-amino-3-fluoro-1-propanol; m.p. 145—147°C, $[\alpha]_D^{26}$ = −59°(0.3% in dimethylformamide). Mass Spectrum: ($M^+$+1) 215. PMR (dmso-$d_6$): $\delta$ 7.42—8.30 (aromatic hydrogens), 4.78 (d, J=4.0 Hz, H—1), 3.8—4.78 (m, $CH_2$), 2.8—3.3 (m, H—2). Analysis: Calculated for $C_9H_{11}N_2O_3F$: C=50.47%, H=5.18%, N=13.08%. Found: C=50.86%, H=5.53%, N=12.70%.

D. D-(*Threo*)-1-*p*-Nitrophenyl-2-Dichloroacetamido-3-Fluoro-1-Propanol

To D-(*threo*)-1-*p*-nitrophenyl-2-amino-3-fluoro-1-propanol prepared as described in Example 1C (18.1 gm.) add 80 ml. of a solution containing methyl dichloroacetate and 7 ml. of methanol. Heat the reaction mixture at 100°C for 4 hours. Monitor the progress of the reaction by thin layer chromatography slowly adding about 1 ml. of triethylamine as the reaction proceeds. Remove the solvents *in vacuo* and wash the resultant residue with petroleum ether (200 ml.). Dissolve the washed residue in chloroform (about 700 ml.), concentrate *in vacuo,* and chromatograph the resultant residue on a column of silica gel (2 kg.) eluting with a mixture of chloroform and ethanol (98:2 by volume). Pour the like fractions containing the desired product as determined by thin layer chromatography and evaporate the combined fractions *in vacuo* to a residue comprising D-(*threo*)-1-*p*-nitrophenyl-2-dichloroacetamido-3-fluoro-1-propanol, yield 19.9 gm. (81.5% theory). Further purified by dissolving the residue in chloroform (200 ml.) (concentrating to a volume of about 100 ml.) and adding petroleum ether (about 300 ml.). Separate the resultant precipitate by filtration to obtain purified D-(*threo*)-1-*p*-nitrophenyl-2-dichloroacetamido-3-fluoro-1-propanol, yield 16.6 gm.; m.p. 103—104.5°C $[\alpha]_D^{26}$ = −23.4° (0.3% in dimethylformamide). Mass Spectrum: ($M^+$325. PMR (dmso-$d_6$): $\delta$ 8.62 (d, J=9.0 Hz, NH), 7.54—8.30 (aromatic hydrogens), 6.5 (s, $CHCl_2$), 6.2 (d, J=4.0 Hz, benzylic OH), 4.0—5.2 (m, $CH_2F$, H—1 and H—2). Analysis: Calculated for $C_{11}H_{11}N_2O_4FCl_2$: C=40.64%, H=3.41%, N=8.62%, Cl=21.81%, F=5.84%. Found: C=41.27%, H=3.74%, N=8.51%, Cl=21.06%, F=6.07%.

## Example 2

### D-(*THREO*)-1-*p*-NITROPHENYL-2-DIFLUOROACETAMIDO-3-FLUORO-1-PROPANOL

To D-(*threo*)-1-*p*-nitrophenyl-2-amino-3-fluoro-1-propanol (0.428 gr., 2 mmol) prepared as for Example 1C add ethanol 5 ml.) and ethyl difluoroacetate (0.5 gm.). Heat the reaction mixture at a reflux temperature for 2 hours, then remove the solvent *in vacuo* and chromatograph the resultant residue on silica gel (50 gm.) eluting with a mixture of chloroform and ethanol (95:5 by volume). Combine the like fractions containing the desired product as determined by thin layer chromatography and evaporate the combined fractions *in vacuo* to a residue (0.47 gm.) comprising D-(*threo*)-1-*p*-nitrophenyl-2-difluoroacetamido-3-fluoro-1-propanol, yield 80% theory; m.p. 97—98°C. Mass Spectrum: $(M^++1)$ 293.PMR $(dmso-d_6)$: $\delta$ 8.8 (d, J=8.0 Hz, NH), 7.49—8.32 (aromatic hydrogens), 6.12 (t, J=54.0 Hz, $CHF_2$), 6.10 (d, J=4.0 Hz, benzylic OH), 4.05—5.05 (m, $CH_2F$, H—1 and H—2).

## Example 3

### D-(*THREO*)-1-*p*-NITROPHENYL-2-TRIFLUOROACETAMIDO-3-FLUORO-1-PROPANOL

In a manner similar to that described in Example 2, treat D-(*threo*)-1-*p*-nitrophenyl-2-amino-3-fluoro-1-propanol in ethanol with ethyl trifluoroacetate. Isolate and purify the resultant product in a manner similar to that described in Example 2 to obtaine D-(*threo*)-1-*p*-nitrophenyl-2-trifluoroacetamido-3-fluoro-1-propanol; m.p. 123—124°C. Mass Spectrum: m/e 152

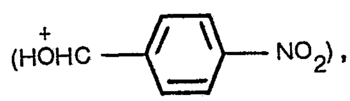

158 ($F_3CCONH^+=CHCH_2F$). PMR $(dmso-d_6)$: $\delta$ 9.5 (d, J=9.0 Hz, NH), 7.5—8.3 (aromatic hydrogens), 6.1 (d, J=4.0 Hz, benzylic OH), 4.1—5.3 (m, $-CH_2$, H—1 and H—2).

## Example 4

### D-(*THREO*)-1-*p*-NITROPHENYL-2-ACETAMIDO-3-FLUORO-1-PROPANOL

*Method A*

To D-(*threo*)-1-*p*-nitrophenyl-2-amino-3-fluoro-1-propanol (0.428 gm., 2 mmol.) prepared as for Example 1C, add ethanol (5 ml.) and then with stirring add acetic anhydride (0.3 gm., 3 mmol.). After 30 minutes at room temperature, remove the solvent *in vacuo,* chromatograph the residue and isolate the product in a manner similar to that described in Example 2 to obtain D-(*threo*)-1-*p*-nitrophenyl-2-acetamido-3-fluoro-1-propanol.

*Method B*

To D-(*threo*)-1-*p*-nitrophenyl-2-amino-3-fluoro-1-propanol (0.428 gm., 2 mmol.) prepared as for Example 1C, add ethanol (5 ml.) and triethylamine (0.202 gm., 2 mmol.). Cool the solution to 5°C with stirring and then add, dropwise, acetyl chloride (0.157 gm., 2 mmol.). After 30 minutes at room temperature, isolate and purify the resultant product in a manner similar to that described in Method A to obtain the title compound.

## Example 5

### D-(*THREO*-1-*p*-NITROPHENYL-2-(R,S-CHLOROFLUOROACETAMIDO)-3-FLUORO-1-PROPANOL

Add triethylamine (0.2 ml.) to a solution of D-(*threo*)-1-*p*-nitrophenyl-2-amino-3-fluoro-1-propanol (prepared as described in Example 1C) (1.1 gm.) and ethyl chlorofluoroacetate (0.5 ml.) in ethanol (14 ml.). Heat at reflux temperature under a blanket of nitrogen for 2 hours. Add an additional amount of ethyl chlorofluoroacetate (0.5 ml.) and continue heating until the reaction is essentially complete as indicated by the absence of starting compound as determined by thin layer chromatography using a mixture of chloroform and methanol (9:1 by volume) as the solvent system. Evaporate the solvents *in vacuo* and chromatograph the resultant residue on silica gel (40 gms.) using chloroform as the eluant. Collect like fractions containing the desired product as determined by thin layer chromatography, evaporate to dryness *in vacuo* to obtain D-(*threo*)-1-*p*-nitrophenyl-2-(*R,S*-chlorofluoroacetamido-3-fluoro-1-propanol (yield 0.97 gm.). Mass Spectrum: ($M^+$ 309, m/e 157, 156, 153, 152, 139, 137, 136, 122, 106, 105, 102, 94, 78, 77, 76, 70, 67, 62, 60, 52, 51 and 50.

Example 6

OTHER D-(THREO)-1-p-NITROPHENYL-2-ACYLAMINO-3-FLUORO-1-PROPANOLS

A. 2-Polyhalogenoacetamido and 2-$\alpha,\alpha$-Dihalogenopropionamido Derivatives

In a manner similar to that described in Example 2, treat D-(*threo*)-1-*p*-nitrophenyl-2-amino-3-fluoro-1-propanol in methanol or ethanol with each of the following substituted-acetic or propionic acid esters:

1) Methyl trichloroacetate,
2) Methyl dibromoacetate,
3) Methyl dichlorofluoroacetate,
4) Methyl $\alpha,\alpha$-difluoropropionate,
5) Methyl $\alpha,\alpha$-dichloropropionate,
6) Methyl chlorodifluoro-acetate.

Isolate and purify each of the resulting products in a manner similar to that described in Example 2 to obtain, respectively,

1) D-(*threo*)-1-*p*-nitrophenyl-2-trichloroacetamido -3-propanol,
2) D-(*threo*)-1-*p*-nitrophenyl-2-dibromoacetamido-3-fluoro-1-propanol,
3) D-(*threo*)-1-*p*-nitrophenyl-2-dichlorofluoroacetamido-3-fluoro-1-propanol,
4) D-(*threo*)-1-*p*-nitrophenyl-2-$\alpha,\alpha$-difluoropropionamido-3-fluoro-1-propanol,
5) D-(*threo*)-1-*p*-nitrophenyl-2-$\alpha,\alpha$-dichloropropionamido-3-fluoro-1-propanol,
6) D-(*threo*)-1-*p*-nitrophenyl-2-chlorodifluoroacetamido-3-fluoro-1-propanol.

B. 2-Monohalogenoalkanoylamino and 2-$\alpha,\beta$-Dihalogenopropionamido Derivatives

In a manner similar to that described in Example 4 (method B), treat D-(*threo*)-1-*p*-nitrophenyl-2-amino-3-fluoro-1-propanol in methanol or ethanol with each of the following substituted acetic or propionic acid chlorides:

7) Fluoroacetyl chloride,
8) Chloroacetyl chloride,
9) Bromoacetyl chloride,
10) Iodoacetyl chloride,
11) Propionyl chloride,
12) $\alpha$-Fluoropropionyl chloride,
13) $\alpha$-Chloropropionyl chloride,
14) $\alpha$-Bromopropionyl chloride,
15) $\alpha$-Iodopropionyl chloride,
16) $\beta$-Chloropropionyl chloride,
17) $\beta$-Bromopropionyl chloride,
18) $\beta$-Iodopropionyl chloride,
19) $\alpha,\beta$-Dichloropropionyl chloride,
20) $\alpha,\beta$-Difluoropropionyl chloride.

Isolate and purify each of the resulting products in a manner similar to that described in Example 2 to obtain, respectively,

7) D-(*threo*)-1-*p*-nitrophenyl-2-fluoroacetamido-3-fluoro-1-propanol,
8) D-(*threo*)-1-*p*-nitrophenyl-2-chloroacetamido-3-fluoro-1-propanol,
9) D-(*threo*)-1-*p*-nitrophenyl-2-bromoacetamido-3-fluoro-1-propanol,
10) D-(*threo*)-1-*p*-nitrophenyl-2-iodoacetamido-3-fluoro-1-propanol,
11) D-(*threo*)-1-*p*-nitrophenyl-2-propionamido-3-fluoro-1-propanol,
12) D-(*threo*)-1-*p*-nitrophenyl-2-$\alpha$-fluoropropionamido-3-fluoro-1-propanol,
13) D-(*threo*)-1-*p*-nitrophenyl-2-$\alpha$-chloropropionamido-3-fluoro-1-propanol,
14) D-(*threo*)-1-*p*-nitrophenyl-2-$\alpha$-bromopropionamido-3-fluoro-1-propanol,
15) D-(*threo*)-1-*p*-nitrophenyl-2-$\alpha$-iodopropionamido-3-fluoro-1-propanol,
16) D-(*threo*)-1-*p*-nitrophenyl-2-$\beta$-chloropropionamido-3-fluoro-1-propanol,
17) D-(*threo*)-1-*p*-nitrophenyl-2-$\beta$-bromopropionamido-3-fluoro-1-propanol,
18) D-(*threo*)-1-*p*-nitrophenyl-2-$\beta$-iodopropionamido-3-fluoro-1-propanol,
19) D-(*threo*)-1-*p*-nitrophenyl-2-$\alpha,\beta$-dichloropropionamido-3-fluoro-1-propanol,
20) D-(*threo*)-1-*p*-nitrophenyl-2-$\alpha,\beta$-difluoropropionamido-3-fluoro-1-propanol.

In a manner similar to that described in Examples 6A and 6B above, other polyhalopropionamido derivatives may be prepared as may the corresponding mixed-polyacyl derivatives of the foregoing.

C. Azidoacetamido and methylsulfonylacetamido derivatives

In a manner similar to that described in Example 2 treat D-(*threo*)-1-*p*-nitrophenyl-2-amido-3-fluoro-1-propanol in methanol or ethanol with methyl or ethyl azidoacetate and methyl or ethyl methylsulfonylacetate to obtain respectively:

D-(*threo*)-1-*p*-nitrophenyl-2-azidoacetamido-3-fluoro-1-propanol, and

D-(*threo*)-1-*p*-nitrophenyl-2-methylsulfonylacetamido-3-fluoro-1-propanol. For the latter compound the following data were obtained: PMR (dmso-$d_6$): $\delta$2.91 (3H, s, $CH_3SO_2CH_2$), 4.04 (2H, s, $CH_3SO_2CH_2$), 6.08 (1H, d, J=5.0 Hz, benzylic OH), 7.86 (4H, center of AA', BB' quartet, aromatic hydrogens).

## Example 7

### D-(THREO)-1-*p*-NITROPHENYL-2-DICHLORODEUTERIOACETAMIDO-3-FLUORO-1-PROPANOL

Add triethylamine (0.7 ml.) to a stirred solution of D-(*threo*)-1-*p*-nitrophenyl-2-dichoro-acetamido-3-fluoro-1-propanol (0.75 gm.) in methyl alcohol-d (i.e., $CH_3OD$) (7 ml.) and stir the solution at room temperature for 18 hours. Concentrate *in vacuo* and dry the resultant residue *in vacuo* over phosphorus pentoxide overnight. Dissolve the residue in methanol (5 ml.) and evaporate to dryness. Repeat this procedure once more. Crystallize the resultant product from an ethyl acetate/*n*-hexane mixture to obtain D-(*threo*)-1-*p*-nitrophenyl-2-dichlorodeuterioacetamido-3-fluoro-1-propanol Yield 0.59 gm.). Mass spectrum: ($M^+$+1) 326. PMR (dmso-$d_6$): $\delta$ 8.6 (d, J=8Hz, NH), 7.96 (ABq, J=10Hz, aromatic hydrogens), 6.15 (d, J=Hz, benzylic OH).

## Example 8

### SODIUM D-(*THREO*)-1-*p*-NITROPHENYL-2-DICHLOROACETAMIDO-3-FLUORO-1-PROPYL HEMISUCCINATE

To D-(*threo*)-1-*p*-nitrophenyl-2-dichloroacetamido-3-fluoro-1-propanol (1.3 gm., 4 mmols) in dioxane (12 ml.) add succinic anhydride (0.92 gm., 8 mmols) and triethylamine (2.4 ml.). Allow the solution to stand at room temperature for 6 hours, then evaporate to a small volume and dissolve the resultant residue in chloroform (200 ml.). Wash the chloroform solution with dilute hydrochloric acid then wash with water; dry over magnesium sulfate, filter and evaporate. Recrystallize the resultant residue from diethylether to obtain D-(*threo*)-1-*p*-nitrophenyl-2-dichloroacetamido-3-fluoro-1-propyl succinate; yield 950 mg. (56% theory).

Dissolve D-(*threo*)-1-*p*-nitrophenyl-2-dichloroacetamido-3-fluoro-1-propanol succinate (850 mg., 2 mmols) in water, add sodium bicarbonate (168 mg., 2 mmols), stir for 15 minutes, filter, add water to the filtrate, then evaporate to a residue comprising sodium D-(*threo*)-1-*p*-nitrophenyl-2-dichloro-acetamido-3-fluoro-1-propyl hemisuccinate; yield 800 mg. PMR ($D_2O$): $\delta$ 2.68 (4H, m, $CH_2$ of the hemi-succinate), 6.1 (1H, d, J=3.0 Hz, benzylic H) 6.28 (1H, s, $CHCl_2$), 7.8 (4H, center of AA, BB quartet, aromatic hydrogens.

## Example 9

### D-(*THREO*)-1-*p*-METHYLSULFONYLPHENYL-2-DICHLOROACETAMIDO-3-FLUORO-1-PROPANOL

A. D-(*Threo*)-1-*p*-Methylsulfonyl-2-Phthalimido-1,3-Propanediol

Suspend thiamphenicol (71 gm., 200 mmol) in water (300 ml.) containing concentrated hydrochloric acid (25 ml.). Stir the reaction mixture at reflux temperature for 6 hours, then evaporate to dryness *in vacuo.* Add toluene (100 ml.) to the resultant residue and evaporate. Repeat this procedure again and dry the residue comprising 1-*p*-methylsulfonylphenyl-2-amino-1,3-propanediol hydrochloride. Stir the foregoing dried residue in toluene (1400 ml.), then add with stirring triethylamine (29.9 gm.) and phthalic anhydride (44 gm.). Heat the reaction mixture at reflux temperature for 6 hours in a flask equipped with a Dean and Stark water remover. Cool the reaction mixture, evaporate the solvent *in vacuo,* then dissolve the resultant residue in ethyl acetate (1.5 liter), wash the ethyl acetate solution with 1N hydrochloric acid (800 ml.), extract the hydrochloric acid washes twice with ethyl acetate (500 ml.) and then combine the ethyl acetate extracts with the original ethyl acetate solution. Dry the combined ethyl acetate solutions over sodium sulfate and evaporate to dryness. Stir the resultant residue in ethanol (300 ml.), then set aside for 2 hours. Separate the resulting solid by filtration, wash the residue with a small quantity of ethanol and dry to give D-(*threo*)-1-*p*-methylsulfonylphenyl-2-phthalimido-1,3-propanediol (yield=35 gm.).

To obtain additional product, evaporate the combined ethanol fitrates *in vacuo,* dissolve the resultant residue in ethyl acetate, wash the ethyl acetate solution with 5% aqueous sodium bicarbonate, dry the ethyl acetate over sodium sulfate, then evaporate *in vacuo.* Triturate the resultant residue with ethanol, and filter to obtain additional D-(*threo*)-1-*p*-methyl-sulfonylphenyl-2-phthalimido-1,3-propanediol (additional yield=7.3 gm); m.p. 209—211°C. Mass Spectrum m/e 190 ($\overset{+}{PhtN}$=$CHCH_2OH$), 186 ($CH_3SO_2C_6H_5CH_2OH$).

B. D-*Threo*)-1-*p*-Methylsulfonylphenyl-2-Phthalimido-3-Fluoro-1-Propanol

Add diethylamine sulfurtrifluoride (25 ml.) dropwise to a stirred suspension of D-(*threo*)-1-*p*-methylsulfonylphenyl-2-phthalimido-1,3-propanediol (66 gm., 0.176 mmol) in tetrahydrofuran (750 ml.) maintained at 0°C with external cooling. After the addition is complete, stir for 30 minutes at 0°C and then for 6 hours at room temperature. Set aside in a refrigerator overnight. Remove the solvent by evaporation *in vacuo* and chromatograph the resultant residue on a silica gel column (4 kg.) eluting with a mixture of chloroform and ethanol (99:1 by volume). Combine like fractions containing the desired product as determined by thin layer chromatography and evaporate the combined fractions *in vacuo* to give a residue of D-(*threo*)-1-*p*-methylsulfonyl-2-phthalimido-3-fluoro-1-propanol, yield 24 gm.; m.p. 166—168°C. Mass Spectrum: m/e 192 (Pth $\overset{+}{N}=CHCH_2F$), 186 ($CH_3SO_2C_6H_5\overset{+}{C}H_2OH$).PMR (dmso-$d_6$): $\delta$ 7.6—8.3 (aromatic hydrogens), 6.1 (d, J=4.0 Hz, benzylic OH), 3.95—5.4 (m, H—1, H—2 and $CH_2F$), 3.23 (s, $SO_2CH_3$).

C. D-(*Threo*)-1-*p*-Methylsulfonyl-phenyl-2-Amino-3-Fluoro-1-Propanol

Add hydrzine hydrate (99%, 0.16 gm.) to a solution of D-(*threo*)-1-*p*-methylsulfonylphenyl-2-phthalimido-3-fluoro-1-propanol (1.14 gm., 3 mmol) in ethanol (20 ml.). Heat at reflux temperature for 4 hours, cool to room temperature and remove the resultant solids by filtration. Evaporate the filtrate to a residue comprising D-(*threo*)-1-*p*-methylsulfonylphenyl-2-amino-3-fluoro-1-propanol.

D. D-(*Threo*)-1-*p*-Methylsulfonylphenyl-2-Dichloroacetamido-3-Fluoro-1-Propanol

Dissolve the D-(*threo*)-1-*p*-methylsulfonylphenyl-2-amino-3-fluoro-1-propanol prepared in Example 9C in methanol (5 ml.) and add methyl dichloroacetate (1 gm.). Heat the reaction mixture at reflux temperature for 2 hours, remove the solvents by evaporation *in vacuo* and extract the resultant residue with a mixture of chloroform and ethanol (75:25 by volume) (100 ml.). Evaporate the organic solvent extracts *in vacuo* and chromatograph the resultant residue on a silica gel colum (90 gm.) eluting with a mixture of chloroform and ethanol (93:7 by volume). Combine the like eluates containing the desired product as determined by thin layer chromatography and evaporate *in vacuo* to a residue of D-(*threo*)-1-*p*-methylsulfonylphenyl-2-dichlorc acetamido-3-fluoro-1-propanol, yield 0.5 gm.; m.p. 146—147°C. Mass Spectrum: $M^+$ 358. PMR (dmso-$d_6$): $\delta$ 8.3 (d, J=8.0 Hz, NH), 7.18—7.75 (aromatic hydrogens), 6.43 (s, $COCHCl_2$), 6.07 (d, J=4.0 Hz, benzylic OH), 3.9—5.1 (m, H—1, H—2 and —$CH_2F$), 3.14 (s, $SO_2CH_3$).

## Example 10

D-(*THREO*)-1-*p*-METHYLSULFONYLPHENYL-2-DIFLUOROACETAMIDO-3-FLUORO-1-PROPANOL

Dissolve the D-(*threo*)-1-*p*-methylsulfonylphenyl-2-amino-3-fluoro-1-propanol prepared in Example 9C in ethanol (5 ml.) and add ethyl difluoroacetate (1 gm.). Heat the reaction mixture at reflux temperature for 2 hours, remove the solvents by evaporation *in vacuo* and extract the resultant residue with a mixtrue of chloroform and ethanol (75:25 by volume; 100 ml.). Evaporate the combined extracts *in vacuo* and chromatograph the resultant residue on silica gel (90 gm.) eluting with a mixture of chloroform:ethanol (93:7). Combine the like fractions containing the desired product as determined by thin layer chromatography and evaporate *in vacuo* to obtain D-(*threo*)-1-*p*-methylsulfonylphenyl-2-difluoroacetamido-3-fluoro-1-propanol, m.p. 141—142°C. Mass Spectrum: ($M^+$+1) 326. PMR (dmso-$d_6$): $\delta$ 8.88 (d, J=8.0 Hz, NH), 7.5—8.1 (aromatic hydrogens), 6.2 (t, J=54 Hz, $COCHF_2$), 6.07 (d, J=4.0 Hz, benzylic OH), 3.7—5.1 (m, H—1, H—2 and $CH_2F$), 3.2 (s, $SO_2CH_3$).

## Example 11

OTHER D-(*THREO*)-1-*p*-METHYLSULFONYLPHENYL-2-ACYLAMINO-3-FLUORO-1-PROPANOLS

A. In a manner similar to that described in Example 9D, treat D-(*threo*)-1-*p*-methylsulfonylphenyl-2-amino-3-fluoro-1-propanol in methanol with methyl trifluoroacetate, and with each of the substituted acetic acid and substituted propionic acid ester starting compounds of Example 6A. Isolate and purify each of the resulting products in a manner similar to that described to obtain, respectively,

1) D-(*threo*)-1-*p*-methylsulfonylphenyl-2-trifluoroacetamido-3-fluoro-1-propanol,
2) D-(*threo*)-1-*p*-methylsulfonylphenyl-2-trichloroacetamido-3-fluoro-1-propanol,
3) D-(*threo*)-1-*p*-methylsulfonylphenyl-2-dibromoacetamido-3-fluoro-1-propanol,
4) D-(*threo*)-1-*p*-methylsulfonylphenyl-2-dichlorofluoroacetamido-3-fluoro-1-propanol,
5) D-(*threo*)-1-*p*-methylsulfonylphenyl-2-$\alpha$,$\alpha$-difluoropropionamido-3-fluoro-1-propanol,
6) D-(*threo*)-1-*p*-methylsulfonylphenyl-2-$\alpha$,$\alpha$-dichloropropionamido-3-fluoro-1-propanol, and
7) D-(*threo*)-1-*p*-methylsulfonylphenyl-2-chlorodifluoroacetamido-3-fluoro-1-propanol.

B. In a manner similar to that described in Example 4 (method B), treat D-(*threo*)-1-*p*-methylsulfonylphenyl-2-amino-3-fluoro-1-propanol in methanol or ethanol with each of the substituted acetic acid and substituted propionic acid chloride starting compounds of Example 6B. Isolate and purify each of

the resulting products in a manner similar to that described to obtain, respectively,

8) D-(*threo*)-1-*p*-methylsulfonylphenyl-2-fluoroacetamido-3-fluoro-1-propanol,
9) D-(*threo*)-1-*p*-methylsulfonylphenyl-2-chloroacetamido-3-fluoro-1-propanol,
10) D-(*threo*)-1-*p*-methylsulfonylphenyl-2-bromoacetamido-3-fluoro-1-propanol,
11) D-(*threo*)-1-*p*-methylsulfonylphenyl-2-iodoacetamido-3-fluoro-1-propanol,
12) D-(*threo*)-1-*p*-methylsulfonylphenyl-2-propionamido-3-fluoro-1-propanol,
13) D-(*threo*)-1-*p*-methylsulfonylphenyl-2-α-fluoropropionamido-3-fluoro-1-propanol,
14) D-(*threo*)-1-*p*-methylsulfonylphenyl-2-α-chloropropionamido-3-fluoro-1-propanol,
15) D-(*threo*)-1-*p*-methylsulfonylphenyl-2-α-bromopropionamido-3-fluoro-1-propanol,
16) D-(*threo*)-1-*p*-methylsulfonylphenyl-2-α-iodopropionamido-3-fluoro-1-propanol,
17) D-(*threo*)-1-*p*-methylsulfonylphenyl-2-β-chloropropionamido-3-fluoro-1-propanol,
18) D-(*threo*)-1-*p*-methylsulfonylphenyl-2-β-bromopropionamido-3-fluoro-1-propanol,
19) D-(*threo*)-1-*p*-methylsulfonylphenyl-2-β-iodopropionamido-3-fluoro-1-propanol,
20) D-(*threo*)-1-*p*-methylsulfonylphenyl-2-α,β-dichloropropionamido-3-fluoro-1-propanol, and
21) D-(*threo*)-1-*p*-methylsulfonylphenyl-2-α,β-difluoropropionamido-3-fluoro-1-propanol.

## Example 12
## D-(*THREO*)-1-*p*-METHYLTHIOPHENYL-2-DICHLOROACETAMIDO-3-FLUORO-1-PROPANOL

A. D-(*Threo*)-1-*p*-Aminophenyl-2-Phthalimido-3-Fluoro-1-Propanol

Dissolve a solution of D-(*threo*)-1-*p*-nitrophenyl-2-phthalimido-3-fluoro-1-propanol (11 gm., 32 mmol) in a solution of dioxan (120 ml.) isopropanol (60 ml.) and water (10 ml.) in a 500 ml. hydrogenation flask. Add 10% palladium-on-charcoal (1 gm.) and concentrated hydrochloric acid (3 ml.) and hydrogenate at a pressure of 3.4 atmospheres (50 psi). Monitor the hydrogenation until all the starting material is converted to the corresponding *p*-aminophenyl derivative ($R_f$=0.49, silica gel thin layer chromatography, dichloro-methane and acetone mixture (90:10 by volume)). Remove the catalyst by filtration and wash the catalyst with isopropanol (20 ml.). Concentrate the combined filtrate and isopropanol washings *in vacuo*, and dissolve the resultant residue in 1 molar sodium hydroxide (30 ml.). Extract the aqueous solution several times with dichloromethane, dry the combined extracts over anhydrous magnesium sulfate and concentrate. Crystallize the resultant residue from anhydrous ethanol:diethyl ether and filter and dry the resultant precipitate to obtain D-(*threo*)-1-*p*-aminophenyl-2-phthalimido-3-fluoro-1-propanol, yield=6.2 gm. (19.7 mmol, 62% theory); m.p. 145.5—147°C. Mass Spectrum: $M^+$314. Analysis: Calculated for $C_{17}H_{15}O_3N_2F$: C=64.97%, H:4.81%, N=8.91%, F=6.05%. Found: C=65.23%, H=4.96%, N=8.79%, F=5.75%.

B. D-(*Threo*)-1-*p*-Methylthiophenyl-2-Phthalimido-3-Fluoro-1-Propanol

To a solution of D-(*threo*)-1-*p*-aminophenyl-2-phthalimido-3-fluoro-1-propanol (3 gm., 9.6 mmol) in methylene chloride (60 ml.) cooled to 0°C, add a solution of nitrosyl chloride in methylene chloride (11 ml., 70 mg. per ml). Stir at 0°C for 20 minutes. Add sodium thiomethylate (0.8 gm.) and ethanol (10 ml.) and stir at room temperature for 18 hours. Add additional sodium thiomethylate (0.2 gm.) and stir for one hour. Filter the reaction mixture and evaporate the filtrate to a residue comprising D-(*threo*)-1-*p*-methylthiophenyl-2-phthalimido-3-fluoro-1-propanol, which is used without further purification in the procedure of Example 12C.

C. D-(*Threo*)-1-*p*-Methylthiophenyl-2-Amido-3-Fluoro-1-Propanol

Dissolve the D-(*threo*)-1-*p*-methylphenyl-2-phthalimido-3-fluoro-1-propanol prepared in Example 12C in absolute ethanol (20 ml.) and add hydrazine hydrate (0.55 ml.). Heat at reflux temperature for 3 hours, then filter.

The filtrate containing D-(*threo*)-1-*p*-methylthiophenyl-2-amino-3-fluoro-1-propanol is used without further purification in the procedure of following Example 12C.

D. D-(*Threo*)-1-*p*-Methylthiophenyl-2-Dichloroacetamido-3-Fluoro-1-Propanol,

Add methyl dichloroacetate (1.4 ml.) to the filtrate containing D-(*threo*)-1-*p*-methylthiophenyl-2-amido-3-fluoro-1-propanol prepared in Example 12C. Heat the reaction mixture at reflux temperature for 2 hours, evaporate *in vacuo* and chromatograph the resultant residue on a silica gel column (90 gm.) eluting with a mixture of methylene chloride, 2-propanol and concentrated ammonium hydroxide (28%) (90:6:0.1 by volume). Combine the like fractions containing the desired product as determined by thin layer chromatography and evaporate the combined like eluates to a residue of D-(*threo*)-1-*p*-methylthiophenyl-2-dichloroacetamido-3-fluoro-1-propanol, yield=1.1 gm. Mass Spectrum $M^+$ 325, 153 ($CH_3SC_6H_4CH_2\overset{+}{O}H$).

## Example 13
## D-(*THREO*)-1-PHENYL-2-DICHLORO-ACETAMIDO-3-FLUORO-1-PROPANOL

A. D-(*Threo*)-1-Phenyl-2-Phthalimido-3-Fluoro-1-Propanol

To a solution of D-(*threo*)-1-*p*-aminophenyl-2-phthalimido-3-fluoro-1-propanol (800 mg.) prepared as described in Example 12A in methylene chloride (40 ml.) cooled to 0°C, add a solution of nitrosyl chloride in methylene chloride (3 ml., 70 mg. per ml.). Stir at 0°C for 20 minutes, then separate the resultant precipitate by filtration and wash with methylene chloride to obtain the diazonium chloride slat from D-(*threo*)-1-*p*-amonophenyl-2-phthalimido-3-fluoro-1-propanol.

Dissolve the foregoing diazonium chloride salt in cold water (6 ml.) and quickly add the solution to 50% aqueous hypophosphorus acid (1.6 gm.) which has been previously cooled in a water bath. Allow the reaction mixture to warm to 25°C, then stir for 3 hours at 25°C. Extract the reaction mixture with methylene chloride (a total of 75 ml.), wash the combined methyl chloride extracts with dilute aqueous sodium bicarbonate, dry the methylene chloride solution over anhydrous magnesium sulfate, and evaporate to a residue comprising D-(*threo*)-1-*p*-phenyl-2-phthalimido-3-fluoro-1-propanol (yield 0.7 gm.), which is used without further purification in the procedure of Example 13B.

B. D-(*Threo*)-1-Phenyl-2-amino-3-Fluoro-1-Propanol

Disolve the D-(*threo*)-1-phenyl-2-phthalimido-3-fluoro-1-propanol prepared in Example 13A in absolute ethanol (10 ml.), add hydrazine hydrate (0.12 ml.) and heat at reflux temperature for 3 hours, then filter, and evaporate the filtrate to a residue comprising D-(*threo*)-1-phenyl-2-amino-3-fluoro-1-propanaol, which is used without further purification in the procedure of following Example 13C.

C. D-(*Threo*)-1-Phenyl-2-Dichloroacetamido-3-Fluoro-1-Propanol

Dissolve the D-(*threo*)-1-phenyl-2-amino-3-fluoro-1-propanol prepared in Example 13B in methanol (6 ml.), add methyl dichloroacetate (1.4 ml.), and heat at reflux temperature, keeping the reaction mixture at pH 8 by the addition of triethylamine. Continue refluxing until all the starting material has been consumed as evidenced by thin layer chromatography on silica gel using a mixture of chloroform and ethanol (97:3 by volume) as solvent, then concentrate the reaction mixture. Purify the resultant residue by chromatography on a silica gel column (2.8 × 30 cm.) eluting with a mixture of chloroform and ethanol (99:1 by volume), taking 4 ml. fractions, 2 minutes per tube. Combine the like fractions containing the desired product as determined by thin layer chromatography and evaporate to a residue comprising D-(*threo*)-1-*p*-phenyl-2-dichloracetamido-3-fluoro-1-propanol (yield 0.4 gm.) (1.43 mmol, 56% theory). Mass Spectrum: 280 ($M^+$+1), 282 ($M^+$+3), 107 ($\overset{+}{HO}$=CH-phenyl). PMR(dmso-$d_6$): $\delta$ 5.86 (benzylic OH), 6.51 $Cl_2CH$—, 7.31 (phenyl).

## Example 14
## D-(*THREO*)-1-*p*-METHYLSULFINYLPHENYL-2-DICHLORO-ACETAMIDO-3-FLUORO-1-PROPANOL

To a solution of D-(*threo*)-1-*p*-methylthiophenyl-2-dichloroacetamido-3-fluoro-1-propanol (compound of Example 12) (910 mg., 2.8 mmol) in 95% dioxan (10 ml.) at 0°C, add sodium metaperiodate (600 mg.) and stir for 30 minutes. Destroy any excess sodium metaperiodate by addition of ethylene glycol until the reaction mixture is negative to starch-iodide paper. Add dioxan (twice the volume of the reaction mixture) and filter the resultant precipitated salts. Evaporate the dioxan filtrate and to the resultant residue add a mixture of chloroform and ethanol (3:1 by volume; 50 ml.), then filter the resultant solid, and wash with the mixture of chloroform and ethanol (3:1). Combine the filtrate and washings, evaporate and chromatograph the resultant residue on a 15 gm. silica gel column (1.8 × 30 ml.) eluting with a mixture of chloroform and ethanol (99:1 by volume). Combine the like fractions containing the desired product as determined by thin layer chromatography and evaporate to a residue comprising D-(*threo*)-1-*p*-methylsulfinylphenyl-2-dichloroacetamido-3-fluoro-1-propanol (yield 247 mg.). Mass Spectrum: 342 ($M^+$+1), 344 ($M^+$+3), 169 ($HO=CHC_6H_4SOCH_3$). PMR(dmso-$d_6$): $\delta$ 5.98 (d, J=3.0 Hz, benzylic OH), 6.45 (s, $Cl_2CHCO$—), 7.58 (s, aromatic) 2.67 (s, $CH_3SO_2$).

## Example 15
## D-(*THREO*)-*p*-NITROPHENYL-2-DICHLORO-ACETAMIDO-3-FLUORO-1-PROPANOL

Stir a mixture of D-(*threo*)-1-*p*-dichloromethyl-4-paranitrophenyl-1-hydroxymethyl-$\Delta^2$-oxazoline (1 gm.), lithium fluoride (0.5 gm.), boron trifluoride etherate (0.1 gm.) and liquid hydrogen fluoride in a teflon-lined bomb for 24 hours. Evaporate off the gaseous hydrogen fluoride into a suitable trap, then dissolve the resultant residue in chloroform and wash the chloroform solution twice with water, then dry over magnesium sulfate and evaporate. Chromatograph the resultant residue in silica gel using chloroform as the eluant. Collect like fractions containing the desired product as determined by thin layer chromatography and evaporate *in vacuo* to obtain a residue comprising D-(*threo*)-1-para-nitrophenyl-2-dichloroacetamido-3-fluoro-1-propanol.

Example 16

D-(*THREO*)-1-*p*-METHYLSULFONYLPHENYL-2-DIFLUOROACETAMIDO-3-FLUORO-1-PROPANOL GLYCINATE TRIFLUOROACETIC ACID SALT

Dissolve D-(*threo*)-1-*p*-methylsulfonylphenyl-2-difluoroacetamido-3-fluoro-1-propanol prepared as in Example 10 (1.29 gm.), *N-p*-methoxybenzyloxycarbonyl glycine (1.1 gm.), N,N-dicyclohexylcarbodiimide (1.1 gm.) and pyridine (0.5 ml.) in acetonitrile (50 ml.). Stir for 4 hours, then evaporate *in vacuo* at 50°C and chromatograph the resultant residue over silica gel (200 mg.) eluting with 1% by volume of ethanol in chloroform. Collect like fractions containing pure D-(*threo*)-1-*p*-methylsulfonylphenyl-2-difluoroacetamido-3-fluoro-1-propanol *N-p*-methoxybenzyloxycarbonylglycinate as determined by thin layer chromatography and evaporate. Dissolve the resultant residue under anhydrous conditions in trifluoroacetic acid (20 ml). Add diethyl ether (100 ml.) to the solution and separate the resultant precipitate by filtration. Dissolve the precipitate in water, filter and lyophilize the aqueous solution to obtain D-(*threo*)-1-*p*-methylsulfonylphenyl-2-difluoroacetamido-3-fluoro-1-propanol glycinate trifluoroacetic acid salt, yield 1.23 gm.; PMR (dmso-$d_6$): $\delta$ 7.84 (ABq, J=8Hz, aromatic hydrogen), 6.25 (d, J=3Hz, H—1), 6.05 (t, J=53Hz, $CH_2F$), 4.07 (s, glycyl $CH_2$), 3.17 (s, $SO_2CH_3$).

Example 17

D-(*THREO*)-1-*p*-METHYLSULFONYLPHENYL-2-DIFLUOROACETAMIDO-3-FLUORO-1-PROPANOL ORNITHATE TRIFLUOROACETIC ACID SALT

Treat D-(*threo*)-1-*p*-methylsulfonylphenyl-2-difluoroacetamido-3-fluoro-1-propanol in a manner similar to that described in Example 16, but use di-*N-p*-methoxybenzyloxycarbonyl ornithine (2.3 gm.) instead of *p*-methoxybenzyloxycarbonyl glycine to obtain D-(*threo*)-1-*p*-methylsulfonylphenyl-2-difluoroacetamido-3-fluoro-1-propanol ornithate trifluoroacetic acid salt, yield 0.84 gm., PMR (dmso-$d_6$): $\delta$=7.87 (ABq, J=8Hz, aromatic hydrogen), 6.19 (d, J=3Hz, H—1), 6.07 (t, J=53Hz, $CH_2F$), 3.2 (s, $SO_2CH_3$), 1.92 (broad, ornithine $CH_2$—$CH_2$), 3.0 (broad, ornithine *CH*).

Example 18

THE SULFURIC ACID SALTS OF D-(-*THREO*)-1-*p*-METHYLSULFONYLPHENYL-2-DIFLUOROACETAMIDO-3-FLUORO-1-PROPANOL GLYCINATE AND OF D-(*THREO*)-1-*p*-METHYLSULFONYL-2-DIFLUOROACETAMIDO-3-FLUORO-PROPANOL ORNITHATE

(1) Dissolve the trifluoroacetic acid salt product of Example 16 in tetrahydrofuran and add 0.5 molar equivalents of sulfuric acid. Evaporate the tetrahydrocuran *in vacuo,* then re-dissolve the resultant residue in tetrahydrofuran and evaporate again. Repeat this procedure until all the trifluoroacetic acid is displaced by sulfuric acid as evidenced by NMR data to obtain D-(*threo*)-1-*p*-methylsulfonylphenyl-2-difluoroacetamido-3-fluoro-1-propanol glycinate sulfuric acid salt.

(2) Treat the trifluoroacetic acid salt of Example 17 in a manner similar to that described above to obtain D-(*threo*)-1-*p*-methylsulfonylphenyl-2-difluoroacetamido-3-fluoro-1-propanol ornithate sulfuric acid salt.

Example 19

D-(*THREO*)-1-*p*-METHYLSULFONYLPHENYL-2-DIFLUOROACETAMIDO-3-FLUORO-1-PROPANOL LYSINATE TRIFLUOROACETIC ACID SALT

Treat D-(*threo*)-1-*p*-methylsulfonylphenyl-2-difluoroacetamido-3-fluoro-1-propanol in a manner similar to that described in Example 16, but use di-N-*p*-methoxybenzyloxycarbonyl lysine (3.06 gm.) instead of *p*-methoxybenzyloxycarbonyl glycine to obtain D-(*threo*)-1-*p*-methylsulfonylphenyl-2-difluoroacetamido-3-fluoro-1-propanol lysinate trifluoroacetic acid salt, yield 3.1 gm., PMR (dmso-$d_6$): $\delta$=7.81 (ABq, J=8Hz, aromatic hydrogens), 6.19 (d, J=3Hz, H—1), 6.08 (t, J=53Hz, $CH_2F$), 3.23 (s, $SO_2CH_3$), 2.90 (broad, lysine *CH*), 1.90 (broad, lysine $CH_2$). As previously mentioned, the compounds of the general formula I exhibit antibacterial activity and are generally broad-spectrum antibiotics useful in the treatment of gram-negative, gram-positive and rickettsial infections and may also be cidal against bacteria resistant to the corresponding 3-hydroxy analogs, for instance chloramphenicol. In this connection, the provision of compounds lacking the 3-primary hydroxy function is particularly useful where the organism in question inactivates by a mechanism involving *O*-acetylation of the primary hydroxy function.

The antibacterial activity and toxicity of the compounds of the invention may be illustrated with reference to data obtained with the following compounds:

Compound A: D-(*threo*)-1-*p*-nitrophenyl-2-dichloroacetamido-3-fluoro-1-propanol,

Compound B: D-(*threo*)-1-*p*-methylsulfonylphenyl-2-dichloroacetamido-3-fluoro-1-propanol,

Compound C: D-(*threo*)-1-*p*-methylsulfonylphenyl-2-difluoroacetamido-3-fluoro-1-propanol.

In standard dilution tests, Compounds A to C exhibited *in vitro* activity against gram-positive bacteria (such as *Staphlococcus aureus)* and against gram-negative bacteria (such as *Escherichia coli,*

*Hemphilus* influenzae, *Klebsiella, Salmonella, Shigella, Proteus, Enterobacter,* and *Serratia* including some bacteria resistant to the 3-hydroxy analog antibiotics.

The *in vivo* activity of the Compounds A to C were determined in mice using mouse protection tests in which the compounds were administered orally about one hour after infection and the mice then observed for 7 days for survival. Results were expressed as $PD_{50}$ valuers and are given in Table A below. For comparison, results obtained for chloroamphenicol (Compound D) and thiamphenicol (compound E) are also included.

TABLE A

*In vivo activity*

| *Bacterial strain* | *$PD_{50}$ (mg/kg)* | | | | |
|---|---|---|---|---|---|
| | A | B | C | D | E |
| *Enterobacter* | | | | | |
| —Charlott | 130 | 25 | 20 | 350 | 300 |
| *Providencia* | | | | | |
| 0828508 | 70 | 55 | 30 | 220 | 200 |
| *Serratia* | | | | | |
| —Brooke 1 | 120 | 15 | 30 | 230 | >200 |
| *Staphylococcus* | | | | | |
| 0413717 | 75 | 45 | 45 | 160 | 160 |

From Table A it will be seen that Compound A, the 3-desoxy-3-fluoro derivative of chloroamphenicol, was significantly more active than chloroamphenicol itself (Compound D). Similarly, Compound B, the 3-desoxy-3-fluoro derivative of thiamphenicol was significantly more active than thiamphenicol itself (Compound E) as was Compound C the 2-difluoroacetamido analog of Compound B.

Toxicity data for Compounds A to C are given in Table B below. The acute toxicity determinations were carried out with groups of five to seven male CF—1 mice. $LD_{50}$ values were calculated on the basis of survivors found 18 and 48 hours after dosing.

TABLE B

*Acute Toxicity*

| Compound | | $LD_{50}$ (mg/kg) (Oral) |
|---|---|---|
| A | | 1650 |
| B | more than | 3000 |
| C | | 880 |

In their use as antibacterial agents, the compounds of the general formula I may be used alone or together with other antibiotics and their use may include reducing the bacterial count in various environments including laboratory glassware, dental and medical equipment.

The compounds of the general formula I find their prime use however in eliciting an antibacterial response in a warm-blooded animal having a susceptible bacterial infection.

They are thus indicated as being useful via the oral or injectible route in the therapeutic treatment

of infective disease including diseases of the upper respiratory tract, e.g., pneumonia, bacterial speticemia, infections of the urogenital and gastrointestinal tracts and in the topical treatment of eye, ear and skin infections.

The compounds of the general formula I may be administered in any suitable form such as in the form of solutions or suspensions for otic and optic use, and may also be administered parenterally via intramuscular injection.

The injectable solution or suspension will usually be administered at a dose of from about 1 mg. to 15 mg. of the compound per kilogram of body weight per day divided into about 2 to about 4 doses. The precise dose depends on the stage and severity of the infection, the susceptibility of the infecting organism to the antibacterial and the individual characteristics of the animal species being treated.

For oral administration, the compounds of the general formula I may be compounded in the form of tablets, capsules, elixers or may even be admixed with animal feed. It is in these dosage forms that the compounds are considered most effective for treating bacterial infections of the gastrointestinal tract causing diarrhoea.

The compounds of the general formula I may also be applied topically in the form of ointments, both hydrophilic and hydrophobic, in the form of lotions which may be aqueous, non-aqueous or of the emulsion type or in the form of creams. Pharmaceutical carriers useful in the preparation of such formulations include such substances as water, oils, greases, polyesters, polyols, and the like.

In general, the topical preparations will contain from about 0.1 to about 3 gm. of the compound of the general formula I per 100 gm. of ointment, cream or lotion. The topical preparations would usually be applied to lesions about 2 to 5 times a day.

Typical pharmaceutical compositions containing a compound of the general formula I as active ingredients are illustrated by way of the following formulation examples:

Formulation Examples'

The following formulation examples are based on the active ingredient being one of the following compounds:

D-(*threo*)-1-*p*-nitrophenyl-2-dichloroacetamido-3-fluoro-1-propanol,

D-(*threo*)-1-*p*-methylsulfonylphenyl-2-dichloroacetamido-3-fluoro-1-propanol, or

D-(*threo*)-1-*p*-methylsulfonylphenyl-2-difluoroacetamido-3-fluoro-1-propanol; however equivalent quantities of other compounds of the formula I may be used.

Formulation 1

*Oral Suspension*

| | mg-ml |
|---|---|
| Active ingredient, micronized | 20.0 |
| Sorbitol solution, USP | 250.0 |
| Methylparaben, USP | 0.5 |
| Propylparaben, USP | 0.1 |
| Propylene glycol, USP | 50.0 |
| Colloidal magnesium aluminum silicate (Veegum HU grade specifically used) | 10.0 |
| Sodium carboxymethylcellulose | 5.0 |
| Surfactant (Pluronic grade F—68 specifically used) | 0.2 |
| Flavoring agent (as required) | |
| Purified water, USP qs ad | 1 ml |

*Manufacturing Procedure:*

Disperse the colloidal magnesium aluminium silicate and sodium carboxymethylcellulose in hot water (80°C). Add the sorbitol solution with stirring, followed by the micronized active ingredient. Add the surfactant prevously dissolved in a portion of hot water (80°C). Cool the batch to 30°C. Dissolve

the methyl and propylparaben in the propylene glycol. Add the solution to the batch. Add the flavouring agent to the batch. Mix until homogenous.

Formulation 2

*Ointment*

| | mg/g |
|---|---|
| Active ingredient, micronized | 20.0 |
| Mineral oil, USP | 50.0 |
| White petroleum, USP qs ad | 1.0 g |

*Manufacturing Procedure:*

Disperse the active ingredient in a portion of the mineral oil and then mill. Heat the white petrolatum and remainder of the mineral oil to 65° and mix until uniform. Cool the mixture to 50—55°C with stirring. Add the mineral oil dispersion of the active ingredient with stirring and then allow the resulting ointment to cool to room temperature.

Formulation 3

*Cream*

| | mg/g |
|---|---|
| Active ingredient, micronized | 20.0 |
| White petrolatum, USP | 150.0 |
| Mineral oil, USP | 50.0 |
| Emulsifying agent (a polyethylene glycol monocetyl ether sold under the designation Cetomacrogol 1000 was used) | 22.0 |
| Cetyl alcohol | 40.0 |
| Stearyl alcohol, USP | 40.0 |
| Sodium phosphate, Monobasic | 4.0 |
| Propylene glycol, USP | 50.0 |
| Purified water qs ad | 1 g |

*Manufacturing Procedure*

Dissolve the propylene glycol and monobasic sodium phosphate in the purified water at 80—90°C. Heat the white petrolatum, Cetomacrogol 1000, cetyl alcohol and stearyl alcohol to 70—75°C and mix until uniform. Add the melted wax mixture to the aqueous mixture with stirring and while cooling to 45—50°C. Slurry and mill the active ingredient in a previously retained portion of Cetomacrogol 1000 dissolved in a portion of the water, also previously retained. Add, with stirring, the slurry containing the active ingredient to the mixture of other ingredients and then allow the resulting cream to cool to room temperature.

Formulation 4

*Lotion*

| | mg/g |
|---|---|
| Active ingredient | 20.0 |
| Propylene glycol, USP | 350.0 |
| Alcohol, USP | 350.0 |
| Hydroxypropylcellulose | 2.5 |
| Purified water qs ad | 1 g |

*Manufacturing Procedure:*

Dissolve the active ingredient in the propylene glycol heated to 50—60°C. Cool to 30—35°C. Add the alcohol and purified water with stirring. Disperse the hydroxypropylcellulose with stirring. Cool to room temperature.

Formulation 5

*Injectable Solution*

| | mg/ml |
|---|---|
| Active ingredient | 250.0 |
| Sodium tartrate | 1.0 |
| Tartaric acid | 4.0 |
| N,N-dimethylacetamide | 500.0 |
| Water for Injection qs ad | 1.0 ml |

*Manufacturing Procedure:*

Dissolve the sodium tartrate and tartaric acid in a portion of Water for Injection. Dissolve the active ingredient in the N,N-dimethylacetamide. Mix the solutions and adjust to the required final volume with Water for Injection. Aseptically filter the solution through a sterile teflon millipore membrane having a pore size of 0.22 microns.

Formulation 6

*Sterile Powder*

The sterile powder is intended for reconstitution with Water for Injection or normal saline to give a solution suitable for parenteral use and having a concentration of 100 mg of the active ingredient per ml of solution.

| | |
|---|---|
| Active ingredient, lyophilized: | 1.0 g |

*Manufacturing Procedure:*

Make a suitable slurry of the drug with Water for Injection and then lyophilize it.

Formulation 7

*Capsules*
*(containing 25, 50 or 250 mg per capsule)*

| Item No. | Ingredient | mg/cap | mg/cap | mg/cap |
|---|---|---|---|---|
| 1. | Active ingredient | 25 | 50 | 250 |
| 2. | Lactose, impalpable powder | 222 | 197 | 185 |
| 3. | Corn starch | 50 | 50 | 60 |
| 4. | Magnesium stearate | 3.0 | 3.0 | 5.0 |
| | TOTAL | 300 mg | 300 mg | 500 mg |

*Manufacture Procedure:*

Mix Item Nos. 1, 2 and 3 in a suitable mixer. Mill the mixture to pass through a No. 40 screen (U.S. Standard). Add Item No. 4 and mix for 3—5 minutes. Encapsulate the mixture in two-piece hard gelatin capsules, using a suitable capsulating machine.

Formulation 8

*Tablets*
*(containing 25, 50 or 250 mg/tablet)*

| Item No. | Ingredient | mg/Tab. | mg/Tab. | mg/Tab. |
|---|---|---|---|---|
| 1. | Active ingredient, micronized | 25 | 50 | 250 |
| 2. | Lactose, impalpable powder | 202.0 | 177.0 | 234 |
| 3. | Microcrystalline cellulose | 30.0 | 30.0 | 60.0 |
| 4. | Corn starch (10% paste in Water) | 10.0 | 10.0 | 20.0 |
| 5. | Corn starch | 30.0 | 30.0 | 30.0 |
| 6. | Magnesium stearate | 3.0 | 3.0 | 6.0 |
| | TOTAL | 300 mg. | 300 mg. | 600 mg. |

*Manufacturing Procedure:*

Mix Item Nos. 1, 2 and 3 in a suitable blender. Add Item No. 4 and mix until a damp mass is formed. Mill to pass through a coarse sieve (e.g. a No. 6 U.S. Standard Sieve) to yield granules. Dry the granules for 8—16 hours at 40—50°C. Mill the dried granules to pass through a No. 20 sieve (U.S. Standard). Add Item No. 5 to the milled granules and mix for 5 to 10 minutes. Add Item No. 6 and then mix further for 3—5 minutes. Compress the mixture into tablets using a suitable tablet press.

**Claims for the Contracting States: BE CH DE FR GB IT NL SE**

1. D-(*threo*)-1-aryl-2-acylamido-3-fluoro-1-propanol compounds of the general formula I

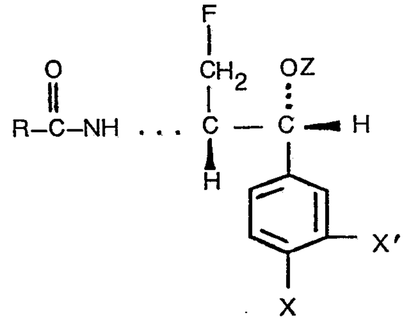

(I),

in which formula:

R represents an alkyl group having one or two carbon atoms which group may be substituted by one or more halogen atoms, or represents azidomethyl, methylsulfonylmethyl or a halodeuteroalkyl group

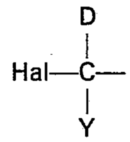

where Y is —$CH_3$, —$CH_2$Hal or Hal, and Hal represents a halogen atom,

X and X′ independently represents a hydrogen or halogen atom, a nitro, cyano or phenyl group which phenyl group may be substituted by halogen or by lower alkyl, lower alkoxy, methylsulfonyl or nitro, or represent a group $R_1(A)_n$ in which group n is zero or 1, A is oxygen, sulfur, carbonyl, sulfinyl or sulfonyl and $R_1$ is lower alkyl, or when A is sulfinyl or sulfonyl then $R_1$ may also be amino or lower alkyl amino, the aforementioned lower alkyl or lower alkoxy groupings having one to three carbon atoms, and

Z is hydrogen or an acyl radical of a carboxylic acid having up to 16 carbon atoms, and the pharmaceutically acceptable salts of the compounds of the formula I where Z is an acyl radical derived from a dicarboxylic acid or amino acid having up to 16 carbon atoms.

2. Compounds as claimed in claim 1, wherein

R represents $(Hal)_2$.CH— or $(Hal)_2$.CD— where Hal independently represents a chlorine or fluorine atom,

X′ is hydrogen and X is nitro or the group $R_1A$ where A is sulfinyl or sulfonyl and $R_1$ is lower alkyl, especially methyl, or when A is sulfonyl then $R_1$ may also be a free amino group, and

Z is hydrogen or an acyl radical derived from an amino acid having up to 12 carbon atoms, preferably an amino acid selected from ornithine, lysine and glycine.

3. A compound as claimed in claim 1, wherein R is $F_2CH$—, $Cl_2CH$— or the corresponding deuterated radicals,

X′ is hydrogen and X is nitro or $CH_3SO_2$—, said compound being selected from the following:

D-(*threo*)-1-*p*-nitrophenyl-2-dichloroacetamido-3-fluoro-1-propanol,

D-(*threo*)-1-*p*-nitrophenyl-2-difluoroacetamido-3-fluoro-1-propanol,

D-(*threo*)-1-*p*-methylsulfonylphenyl-2-dichloroacetamido-3-fluoro-1-propanol,

D-(*threo*)-1-*p*-methylsulfonylphenyl-2-difluoroacetamido-3-fluoro-1-propanol,

and the corresponding 2-difluorodeuterioacetamido- and 2-dichlorodeuterioacetamido analogs of the foregoing compounds.

4. D-(*threo*)-1-*p*-methylsulfonylphenyl-2-difluoroacetamido-3-fluoro-1-propanol in the form of its glycinate, lysinate or ornithate ester as claimed in claims 2 and 3.

5. A process for the preparation of the compounds of the general formula I set forth in claim 1 which process comprises one of the following processes A to D:

A: N-acylating an amine of the general formula II

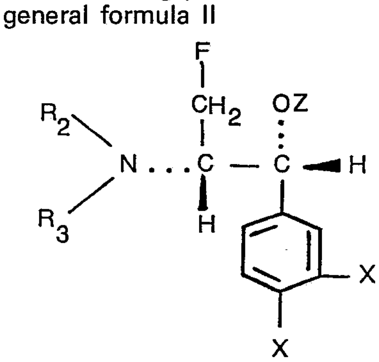

(II),

wherein $R_2$ and $R_3$ are each hydrogen and X, X′ and Z are as defined in formula I, Z being most preferably hydrogen, with a free acid or with a reactive derivative of an acid of the formula R.COOH where R is as defined in claim 1,

B: ring opening, with concomitant fluorination at the 3-position of the propane moiety, of an oxazoline of the general formula IV

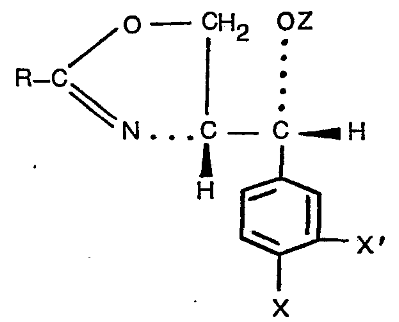

(IV),

wherein R, X, X′ and Z are as defined in claim 1, Z being most preferably hydrogen,

C: oxidising a starting material differing from the desired product of the general formula I in having in one or both of the para- and meta-positions of the phenyl moiety a substituent —$SR_1$ or —$SOR_1$, where $R_1$ is as defined for formula I, to effect conversion of said substituent into —$SOR_1$ and —$SO_2R_1$ respectively, and

D: deuterating a starting material of the general formula I wherein R is

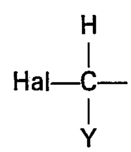

where Y is as defined for formula I, to give the corresponding deutero compound of the formula I where R is

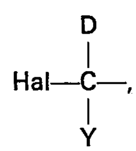

said process selected from processes A to D being followed, if desired, by one or more of the steps of:

(i) esterification or hydrolysis at the 1-hydroxy function, and

(ii) formation of a pharmaceutically acceptable salt where Z is an acyl radical derived from a dicarboxylic acid or an amino acid having up to 16 carbon atoms.

6. A process as claimed in claim 5 process A, wherein the reactive derivative is a lower alkyl ester.

7. A process as claimed in claim 5 process A, which process further comprises the step of preparing the starting material of the general formula II by fluorinating at the 3-position a 1,3-propanediol derivative of the general formula III

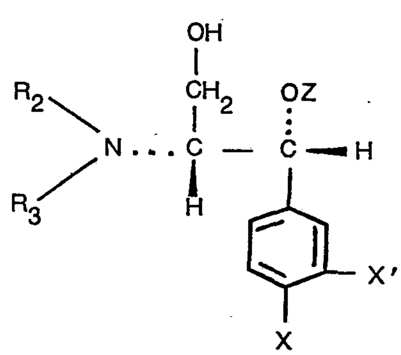

(III),

whereby the 3-hydroxy function is replaced by fluorine, in which formula X, X′ and Z are as defined in claim 1, Z being most preferably hydrogen, and $R_2$, $R_3$ individually or together represent an amino

protecting group, which amino protecting group is subsequently removed.

8. A process as claimed in claim 7, wherein the fluorination is effected with a sulfur trifluoride-secondary amine adduct, preferably diethylamino-sulfur trifluoride.

9. A compound as claimed in any one of claims 1 to 4 for use as a pharmaceutical, especially as an antibacterial.

10. A pharmaceutical composition, especially for use as an antibacterial, comprising as active ingredient a compound as claimed in any one of claims 1 to 4 together with a suitable pharmaceutical carrier.

**Claims for the Contracting State: AT**

1. A process for the preparation of D-(*threo*)-1-aryl-2-acylamido-3-fluoro-1-propanol compounds of the general formula I

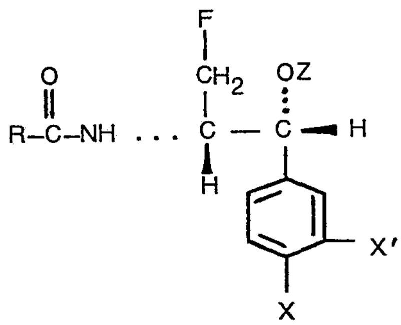

(I),

in which formula:
R represents an alkyl group having one or two carbon atoms which group may be substituted by one or more halogen atoms, or represents azidomethyl, methylsulfonylmethyl or a halodeuteroalkyl group

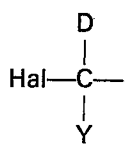

where Y is $-CH_3$, $-CH_2Hal$ or Hal, and Hal represents a halogen atom,
X and X′ independently represent a hydrogen or halogen atom, a nitro, cyano or phenyl group which phenyl group may be substituted by halogen or by lower alkyl, lower alkoxy, methylsulfonyl or nitro, or represent a group $R_1(A)_n$ in which group n is zero or 1, A is oxygen, sulfur, carbonyl, sulfinyl or sulfonyl and $R_1$ is lower alkyl, or when A is sulfinyl or sulfonyl then $R_1$ may also be amino or lower alkyl amino, the aforementioned lower alkyl or lower alkoxy groupings having one to three carbon atoms, and
Z is hydrogen or an acyl radical of a carboxylic acid having up to 16 carbon atoms, and the pharmaceutically acceptable salts of the compounds of the formula I where Z is an acyl radical derived from a dicarboxylic acid or amino acid, having up to 16 carbon atoms, which process comprises one of the following processes A to D:

A: N-acylating an amine of the general formula II

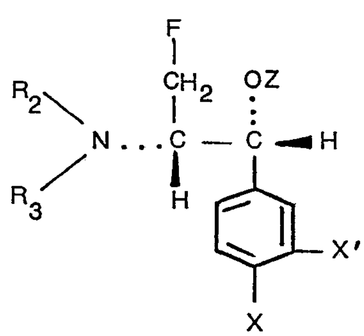

(II),

wherein $R_2$ and $R_3$ are each hydrogen and X, X′ and Z are as defined in formula I, Z being most preferably hydrogen, with a free acid or with a reactive derivative of an acid of the formula R.COOH where R is as defined for formula I,
B: ring opening, with concomitant fluorination at the 3-position of the propane moiety, of an oxazoline of the general formula IV

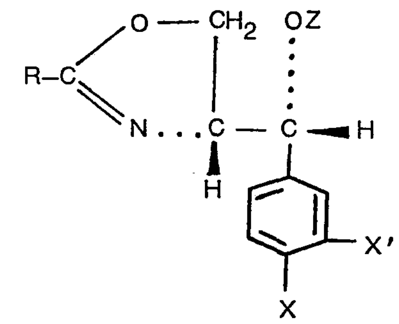

(IV),

wherein R, X, X′ and Z are as defined for formula I, Z being most preferably hydrogen,
C: oxidising a starting material differing from the desired product of the general formula I in having in one or both of the para- and meta-positions of the phenyl moiety a substituent $—SR_1$ or $—SOR_1$, where $R_1$ is as defined for formula I, to effect conversion of the said substituent into $—SOR_1$ and $—SO_2R_1$ respectively, and
D: deuterating a starting material of the general formula I where R is

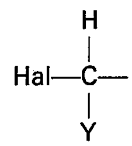

where Y is as defined for formula I, to give the corresponding deutero compound of the formula I where R is

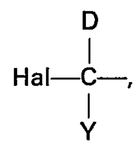

said process selected from processes A to D being followed, if desired, by one or more of the steps of:
(i) esterification or hydrolysis at the 1-hydroxy function, and
(ii) formation of a pharmaceutically acceptable salt where Z is an acyl radical derived from a dicarboxylic acid or an amino acid having up to 16 carbon atoms.

2. A process as claimed in claim 1 process A, characterised in that in the amine starting material of the general formula II, Z and X′ are both hydrogen and X is nitro or the group $R_1A$ where A is sulfinyl or sulfonyl and $R_1$ is lower alkyl, or when A is sulfonyl then $R_1$ may also be a free amino group.

3. A process as claimed in claim 2, characterised in that X is nitro or methylsulfonyl.

4. A process as claimed in claim 1 process A or in claim 2 or claim 3, characterised in that in the reactive derivative of the acid of the formula R.COOH, R represents $(Hal)_2.CH—$ where Hal independently represents a chloro or fluoro atom.

5. A process as claimed in claim 1 process A or in any one of claims 2 to 4, characterised in that the reactive derivative of the acid of the formula R.COOH is a lower alkyl ester.

6. A process as claimed in claim 1 process A or in any one of claims 2 to 5, characterised in that said process further comprises the step of preparing the starting material of the general formula II by fluorinating at the 3-position a 1,3-propanediol derivative of the general formula III

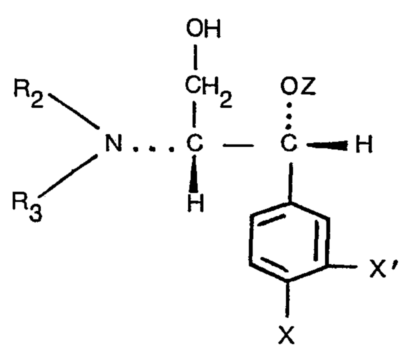

(III),

whereby the 3-hydroxy function is replaced by fluorine, in which formula X, X′ and Z are as defined in

claim 1, Z being most preferably hydrogen, and $R_2$, $R_3$ individually or together represent an amino protecting group, which amino protecting group is subsequently removed.

7. A process as claimed in claim 1 process D, characterised in that deuteration is effected using $CH_3OD$.

8. A process as claimed in any one of the preceding claims, characterised in that a so-obtained compound of the general formula I where Z is hydrogen is esterified with an amino acid, preferably with an amino acid selected from ornithine, lysine and glycine, and the so-obtained ester isolated as such or in the form of an acid addition salt.

9. A process as claimed in claim 8, characterised in that the so-obtained ester is isolated as the sulfate or hydrochloride salt.

10. A process for the preparation of a pharmaceutical composition, characterised in that a compound of the general formula I or a pharmaceutically acceptable salt thereof is brought into a form suitable for therapeutic administration.

**Revendications pour les Etats contractants: BE CH DE FR GB IT NL SE**

1. Composé de D-(thréo)-1-aryl-2-acylamino-3-fluoro-1-propanol de formule générale I:

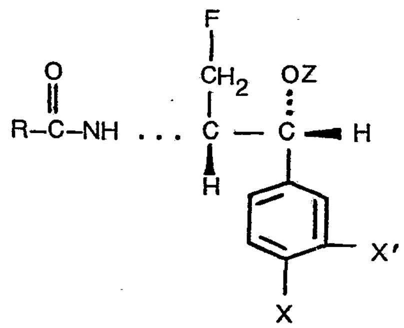

(I)

où

R représente un groupe alcoyle ayant un ou deux atomes de carbone, lequel groupe peut être substitué par un ou plusieurs atomes d'halogène, ou représente un azidométhyle, un méthylsulfonylméthyle ou un groupe halodeutéroalcoyle

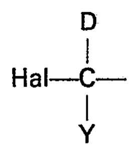

où Y est $—CH_3$, $—CH_2Hal$ ou Hal, et Hal représente un atome d'halogène,

X et X' représentent indépendamment un atome d'hydrogène ou un halogène, un groupe nitro, cyano ou phényle, lequel groupe phényle peut être substitué par un halogène ou par un alcoyle inférieur, un alcoxy inférieur, un méthylsulfonyle ou un groupe nitro, ou représentent un groupe $R_1(A)_m$ dans lequel n est zéro ou 1, A est de l'oxygène, du soufre, un carbonyle, un sulfinyle ou un sulfonyle et $R_1$ est un alcoyle inférieur, ou quand A est un sulfinyle ou un sulfonyle alors $R_1$ peut être aussi un groupe amino ou un groupe amino-alcoyle inférieur, les groupes mentionnés ci-dessus alcoyle inférieur et alcoxy inférieur ayant un à trois atomes de carbone, et

Z est de l'hydrogène ou un radical acyle d'un acide carboxylique ayant jusqu'à 16 atomes de carbone, et les sels acceptables en pharmacie des composés de formule I où Z est un radical acyle dérivé d'un acide dicarboxylique ou d'un amino-acide ayant jusqu'à 16 atomes de carbone.

2. Composé selon la revendication 1, caractérisé en ce que R représente $(Hal)_2$ CH— ou $(Hal)_2$.CD— où Hal représente indépendamment un atome de chlore ou de fluor, X' est de l'hydrogène et X est un groupe nitro ou le groupe $R_1A$ où A est un sulfinyle ou un sulfonyle et $R_1$ est un alcoyle inférieur, en particulier du méthyle, ou quand A est un sulfonyle alors $R_1$ peut aussi être un groupe amino libre, et

Z est de l'hydrogène ou un radical acyle dérivé d'un amino acide ayant jusqu'à 12 atomes de carbone, de préférence un amino-acide choisi parmi l'ornithine, la lysine et la glycine.

3. Composé selon la revendication 1, caractérisé en ce que R est $F_2CH—$, $Cl_2CH—$ ou les radicaux correspondants deutérés, X' est de l'hydrogène et X est un groupe nitro ou $CH_3SO_2—$, ledit composé étant choisi parmi les suivants:

D-(thréo)-1-p-nitrophényl-2-dichloroacétamido-3-fluoro-1-propanol,

D-(thréo)-1-p-nitrophényl-2-difluoroacétamido-3-fluoro-1-propanol,

D-(thréo)-1-p-méthylsulfonylphényl-2-dichloroacétamido-3-fluoro-1-propanol,

D-(thréo)-1-p-méthylsulfonylphényl-2-difluoroacétamido-3-fluoro-1-propanol, et les analogues correspondants 2-difluorodeutérioacétamido et 2-dichlorodeutérioacétamido des composés précédents.

4. D-(thréo)-1-p-méthylsulfonylphényl-2-difluoroacétamido-3-fluoro-1-propanol sous forme de son ester glycinate, lysinate ou ornithate tel que revendiqué dans l'une des revendications 2 et 3.

5. Procédé de préparation des composés de formule générale (I) selon la revendication 1, caractérisé en ce qu'il comprend l'un des procédés suivants A à D:

A: N-acylation d'une amine de formule générale II

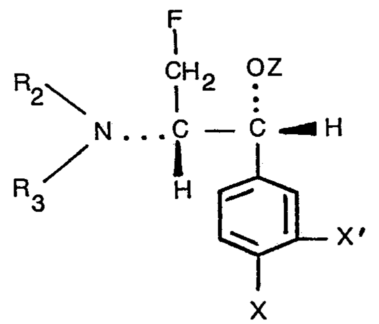

(II)

où $R_2$ et $R_3$ sont chacun de l'hydrogène et X, X' et Z sont tels que définis pour la formule (I), Z étant de préférence de l'hydrogène, avec un acide libre ou avec un dérivé réactif d'un acide de formule R.COOH où R est tel que défini pour la formule (I),

B: ouverture de cycle avec fluoration concomitante en position 3 de la partie propane d'une oxazoline de formule générale (IV)

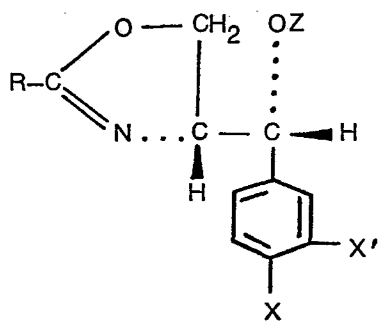

(IV)

où R, X, X' et Z sont tels que définis pour la formule I, Z étant de préférence de l'hydrogène,

C: oxydation d'un matériau de départ différant du produit désiré de la formule générale (I) en ce qu'il a dans une ou dans les deux positions para et méta de la partie phényle un substituant —$SR_1$ ou —$SOR_1$, où $R_1$ est tel que défini pour la formule (I) pour effectuer la conversion desdits substituants en —$SOR_1$ et —$SO_2R_1$, respectivement, et

D: deutération d'un matériau de départ de formule générale (I) où R est

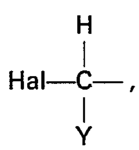

où Y est tel que défini pour la formule (I), pour donner le composé deutéré correspondant de la formule (I) où R est

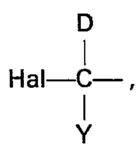

ledit procédé choisi parmi les procédés A à D étant suivi, si on le souhaite, par une ou plus des étapes suivantes:

(i) estérification ou hydrolyse de la fonction 1-hydroxy, et

(ii) formation d'un sel acceptable en pharmacie où Z est un radical acyle dérivé d'un acide dicarboxylique ou d'un amino-acide ayant jusqu'à 16 atomes de carbone.

6. Procédé selon la revendication 5, caractérisé en ce que dans le procédé A, le dérivé réactif est un ester alcoyle inférieur.

7. Procédé selon la revendication 5, caractérisé en ce que le procédé A comprend de plus l'étape de préparer le matériau de départ de formule générale (II) par fluoration en position 3 d'un dérivé 1,3-propanediol de formule générale (III)

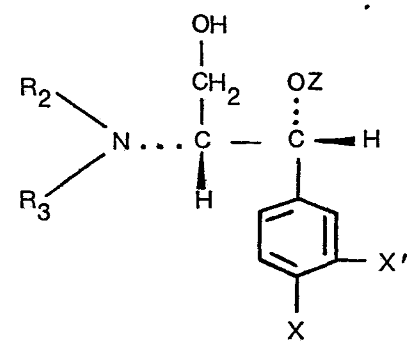

(III)

pour ainsi remplacer la fonction 3-hydroxy par du fluor, formule dans laquelle X, X' et Z sont tels que définis pour la formule (I), Z étant de préférence de l'hydrogène, $R_2$, $R_3$ représentent individuellement ou ensemble un groupe protecteur d'amine, lequel groupe protecteur d'amine est ensuite éliminé.

8. Procédé selon la revendication 7, caractérisé en ce que la fluoration est effectuée avec un ajout d'une amine secondaire sulfotrifluorure, de préférence de la diéthylamine sulfotrifluorure.

9. Composé selon l'une des revendications 1 à 4, caractérisé en ce qu'il est utilisé comme composé pharmaceutique, en particulier comme composé antibactérien.

10. Composition pharmaceutique, utilisée en particulier comme agent antibactérien, caractérisée en ce qu'elle comprend comme ingrédient actif un composé tel que revendiqué dans l'une des revendications 1 à 4 et un support pharmaceutique convenable.

**Revendications pour l'Etat contractant: AT**

1. Procédé de préparation de composés de D-(thréo)-1-aryl-2-acylamido-3-fluoro-1-propanols de formule générale I:

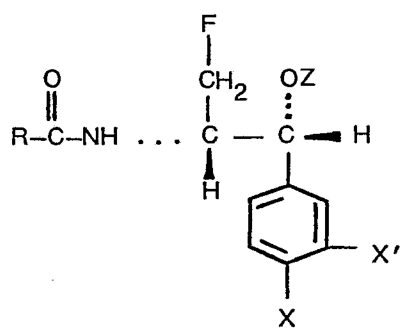

(I)

où

R représente un groupe alcoyle ayant un ou deux atomes de carbone, lequel groupe peut être substitué par un ou plusieurs atomes d'halogène, ou représente un azidométhyle, un méthylsulfonylméthyle ou un groupe halodeutéroalcoyle

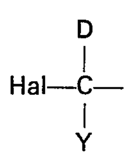

où Y est $-CH_3$, $-CH_2Hal$ ou Hal, et Hal représente un atome d'halogène,

X et X' représentent indépendamment un atome d'halogène ou de l'hydrogène, un groupe nitro, cyano ou phényle, lequel groupe phényle peut être substitué par un halogène ou par un alcoyle inférieur, un alcoxy inférieur, un méthylsulfonyle ou un groupe nitro, ou représentent un groupe $R_1(A)_n$ dans lequel n est zéro ou 1, A est de l'oxygène, du soufre, un carbonyle, un sulfinyle ou un sulfonyle et $R_1$ est un alcoyle inférieur, ou quand A est un sulfinyle ou un sulfonyle alors $R_1$ peut aussi être un groupe amino

ou un groupe amino-alcoyle inférieur, les groupes alcoxy inférieur et alcoyle inférieur mentionnés ci-dessus ayant de 1 à 3 atoms de carbone, et Z est de l'hydrogène ou un radical acyle d'un acide carboxylique ayant jusqu'à 16 atomes de carbone, et les sels acceptables en pharmacie des composés de formule I où Z est un radical acyle dérivé d'un acide dicarboxylique ou d'un amino-acide ayant jusqu'à 16 atomes de carbone, ledit procédé étant caractérisé par l'un des procédés A à D suivants:

A: N-acylation d'une amine de formule générale (II)

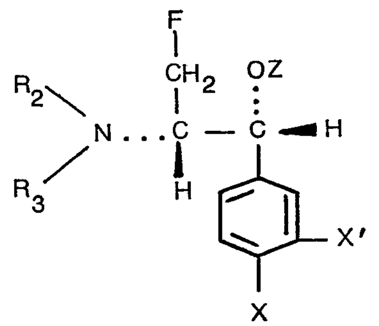

(II)

où $R_2$ et $R_3$ sont chacun de l'hydrogène et X, X' et Z sont tels que définis pour la formule (I), Z étant de préférence de l'hydrogène, avec un acide libre ou avec un dérivé réactif d'un acide de formule R.COOH où R est tel que défini pour la formule (I),

B: ouverture de cycle, avec fluoration concomitante en position 3 de la partie propane d'une oxazoline de formule générale (IV)

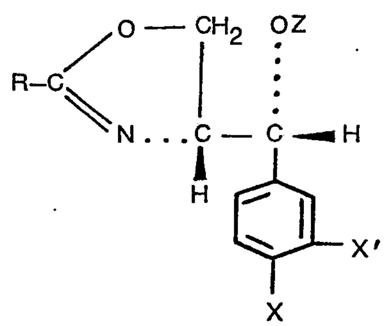

(IV)

où R, X, X' et Z sont tels que définis pour la formule (I), Z étant de préférence de l'hydrogène,

C: oxydation d'un matériau de départ différant du produit désiré de formule générale (I) en ce qu'il a dans une ou dans les deux positions méta et para de la partie phényle un substituant $—SR_1$ ou $—SOR_1$, où R est tel que défini pour la formule (I) pour effectuer la conversion dudit substituant en $—SOR_1$ et $—SO_2R_1$, respectivement,

D: deutération d'un matériau de départ de formule générale (I) où R est

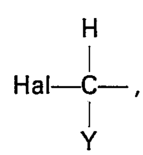

où Y est tel que défini pour la formule (I), pour donner le composé deutéré correspondant de la formule (I) où R est

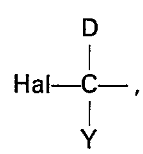

le procédé choisi parmi les procédés A à D étant suivi, si on le souhaite, par une ou plus des étapes suivantes:

(i) estérification ou hydrolyse de la fonction 1-hydroxy, et

(ii) formation d'un sel acceptable en pharmacie où Z est un radical acyle dérivé d'un acide dicarboxylique ou d'un amino acide ayant jusqu'à 16 atomes de carbone.

2. Procédé selon la revendication 1, caractérisé en ce que dans le procédé A, dans le matériau de départ aminé de formule générale (II), Z et X' sont tous les deux de l'hydrogène et X est un groupe nitro ou le groupe $R_1A$ où A est un sulfinyle ou un sulfonyle et $R_1$ est un alcoyle inférieur, ou quand A est un sulfonyle alors $R_1$ peut aussi être un groupe aminé libre.

3. Procédé selon la revendication 2, caractérisé en ce que X est un groupe nitro ou un méthylsulfonyle.

4. Procédé selon l'une des revendications précédentes, caractérisé en ce que dans le procédé A, dans le dérivé réactif de l'acide de formule R.COOH, R représente $(Hal)_2CH$— où Hal représente indépendamment un atome de chlore ou de fluor.

5. Procédé selon l'une des revendications précédentes, caractérisé en ce que, dans le procédé A, le dérivé réactif de l'acide de formule R.COOH est un ester alcoyle inférieur.

6. Procédé selon l'une des revendications précédentes, caractérisé en ce que le procédé A comprend de plus l'étape de préparer le matériau de départ de formule générale (II) par fluoration en position 3 d'un dérivé de 1,3-propanediol de formule générale (III)

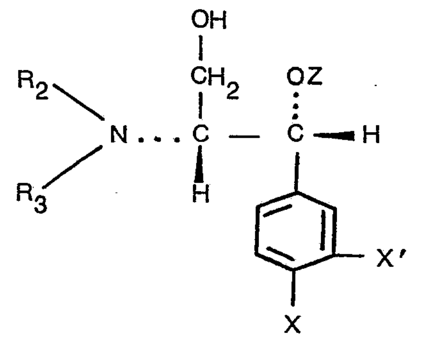

(III)

pour qu'ainsi la fonction 3-hydroxy soit remplacée par du fluor, formule dans laquelle X, X' et Z sont tels que définis pour la formule (I), Z étant de préférence de l'hydrogène, et $R_2$, $R_3$ représentent individuellement ou ensemble un groupe protecteur d'amine, lequel groupe protecteur d'amine est ensuite éliminé.

7. Procédé selon la revendication 1, caractérisé en ce que, dans le procédé D, la deutération est effectuée en utilisant $CH_3OD$.

8. Procédé selon l'une revendications précédentes, caractérisé en ce qu'un composé ainsi obtenu de formule générale (I) où Z est de l'hydrogène est estérifié avec un amino-acide, de préférence avec un amino-acide choisi parmi l'ornithine, la lysine et la glycine, et l'ester ainsi obtenu est isolé tel quel ou sous forme d'un sel d'addition acide.

9. Procédé selon la revendication 8, caractérisé en ce que l'ester ainsi obtenu est isolé sous forme du sel de sulfate ou d'hydrochlorure.

10. Procédé pour la préparation d'une composition pharmaceutique, caractérisé en ce qu'un composé de formule générale (I) ou un sel acceptable en pharmacie de celui-ci est utilisé sous une forme convenable pour l'administration thérapeutique.

**Patentansprüche für die Vertragsstaaten: BE CH DE FR GB IT NL SE**

1. D-(threo)-1-Aryl-2-acylamido-3-fluoro-1-propanol-Verbindung der allgemeinen Formel (I)

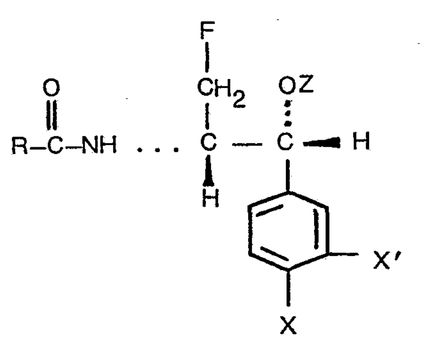

(I),

in der
R für eine Alkyl-Gruppe mit einem oder zwei Kohlenstoff-Atomen, die durch ein oder mehrere Halogen-Atome substituiert sein kann, oder für Azidomethyl, Methylsulfonylmethyl oder eine Halogenodeutero-alkyl-Gruppe steht,

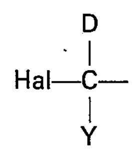

in der Y $—CH_3$, $—CH_2Hal$ oder Hal ist und Hal ein Halogen-Atom bezeichnet, steht, X und X′ unabhängig voneinander für ein Wasserstoff- oder Halogen-Atom, eine Nitro-, Cyano- oder Phenyl- Gruppe, welch letztere durch Halogen oder durch niederes Alkyl, niederes Alkoxy, Methylsulfonyl oder Nitro substituiert sein kann, oder für eine Gruppe $R_1(A)_n$ stehen, in der n 0 oder 1 ist, A Sauerstoff, Schwefel, Carbonyl, Sulfinyl oder Sulfonyl ist und $R_1$ niederes Alkyl ist oder in dem Fall, in dem A Sulfinyl oder Sulfonyl ist, auch Amino oder niederes Alkylamino sein kann, wobei die vorbezeichneten niederen Alkyl- oder niederen Alkoxy-Gruppen ein bis drei Kohlenstoff-Atome besitzen, und

Z Wasserstoff oder ein Acyl-Rest einer Carbonsäure mit bis zu 16 Kohlenstoff-Atomen ist, sowie die pharmazeutisch unbedenklichen Salze der Verbindungen der Formel (I), in denen Z ein von einer Dicarbonsäure oder Aminosäure mit bis zu 16 Kohlenstoff-Atomen abgeleiteter Acyl-Rest ist.

2. Verbindungen nach Anspruch 1, dadurch gekennzeichnet, daß

R $(Hal)_2CH—$ oder $(Hal)_2CD—$ bezeichnet, worin die beiden Hal gleich oder verschieden sein können und für ein Fluor- oder Chlor-Atom stehen,

X′ Wasserstoff ist und

X Nitro oder die Gruppe $R_1A$ ist, worin A Sulfinyl oder Sulfonyl ist und $R_1$ niederes Alkyl, insbesondere Methyl, ist oder, in dem Fall, in dem A Sulfonyl ist, auch eine freie Amino-Gruppe sein kann, und

Z Wasserstoff oder ein von einer Aminosäure mit bis zu 12 Kohlenstoff-Atomen, vorzugsweise einer aus Ornithin, Lysin, und Glycin ausgewählten Aminosäure, abgeleiteter Acyl-Rest ist.

3. Verbindung nach Anspruch 1, dadurch gekennzeichnet, daß R $F_2CH—$, $Cl_2CH—$ oder einer der entsprechenden deuterierten Reste ist,

X′ Wasserstoff ist und

X Nitro oder $CH_3SO_2—$ ist,

wobei die Verbindung ausgewählt ist aus

D-(threo)-1-p-Nitrophenyl-2-dichloroacetamido-3-fluoro-1-propanol,

D-(threo)-1-p-Nitrophenyl-2-difluoroacetamido-3-fluoro-1-propanol,

D-(threo)-1-p-Methylsulfonylphenyl-2-dichloroacetamido-3-fluoro-1-propanol,

D-(threo)-1-p-Methylsulfonylphenyl-2-difluoroacetamido-3-fluoro-1-propanol,

sowie den entsprechenden 2-Difluorodeuterioacetamido und 2-Dichlorodeuterioacetamido-Analoga der vorbezeichneten Verbindungen.

4. D-(threo)-1-p-Methylsulfonylphenyl-2-difluoroacetamido-3-fluoro-1-propanol in Form seines Glycinat-, Lysinat- oder Ornithinat-Esters nach Anspruch 2 und 3.

5. Verfahren zur Herstellung der Verbindungen der allgemeinen Formel (I) nach Anspruch 1, dadurch gekennzeichnet, daß es eines der nachstehenden Verfahren A bis D umfaßt:

A: N-Acylierung eines Amins der allgemeinen Formel (II)

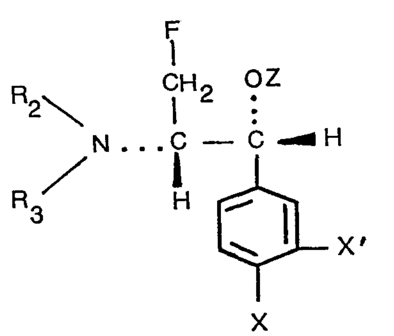

(II),

in der

$R_2$ und $R_3$ jeweils Wasserstoff sind,

X, X′ und Z die für die Formel (I) definierten Bedeutungen haben, wobei besonders bevorzugt Z Wasserstoff ist, mit einer freien Säure oder einem reaktionsfähigen Derivat einer Säure der Formel R—COOH, in der R die für die in Anspruch 1 angegebene Bedeutung hat;

B: Ringöffnung, verbunden mit einer Fluorierung in der 3-Stellung der Propan-Struktureinheit, eines Oxazolins der Allgemeinen Formel (IV)

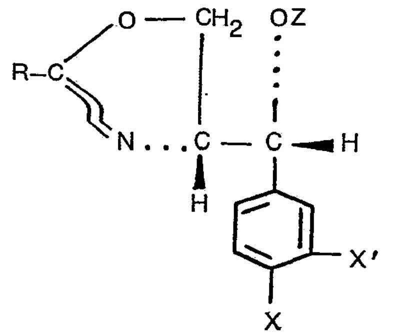

(IV),

in der R, X, X' und Z die in Anspruch 1 definierten Bedeutungen haben wobei besonders bevorzugt Z Wasserstoff ist;

C: Oxidation eines Ausgangsmaterials, das sich von dem angestrebten Produkt der allgemeinen Formel (I) dadurch unterscheidet, daß es in einer oder beiden der p- und m-Stellungen der Phenyl-Struktureinheit einen Substituenten —$SR_1$ oder —$SOR_1$ trägt, worin $R_1$ die für die Formel (I) definierte Bedeutung hat, so daß eine Umwandlung der bezeichneten Substituenten in —$SOR_1$ bzw. —$SO_2R_1$ bewirkt wird; und

D: Deuterierung eines Ausgangsmaterials der Formel (I), in dem R

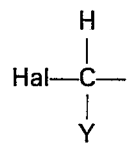

ist, worin Y die für die Formel (I) angegebene Bedeutung hat, unter Bildung der entsprechenden Deuteroverbindung

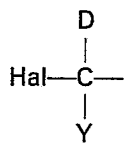

wobei auf das aus den Verfahren A bis D ausgewählte Verfahren, falls erwünscht, eine oder mehrere der Stufen

(i) Veresterung oder Hydrolyse an der 1-Hydroxy-Funktion und

(ii) Bildung eines pharmazeutisch unbedenklichen Salzes, in dem Z ein von einer Dicarbonsäure oder einer Aminosäure mit bis zu 16 Kohlenstoff-Atomen abgeleiteter Acyl-Rest ist.

6. Verfahren nach Anspruch 5, Verfahren A, dadurch gekennzeichnet, daß das reaktionsfähige Derivat ein niederer Alkylester ist.

7. Verfahren nach Anspruch 5, Verfahren A, dadurch gekennzeichnet, daß das Verfahren weiterhin die Stufe der Herstellung des Ausgangsmaterials der allgemeinen Formel (II) durch Fluorierung in 3-Stellung eines 1,3-Propandiol-Derivats der allgemeinen Formel (III)

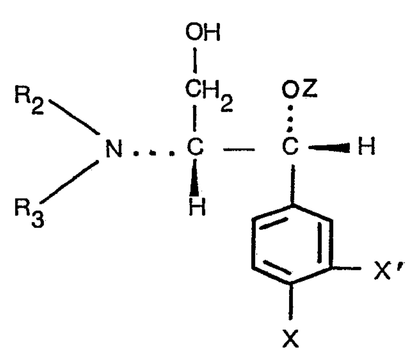

(III),

in der X, X' und Z die in Anspruch 1 definierten Bedeutungen haben, wobei besonders bevorzugt Z Wasserstoff ist, und $R_2$ und $R_3$ einzeln oder gemeinsam Amino-Schutzgruppen bezeichnen, wodurch die 3-Hydroxy-Funktion durch Fluor ersetzt wird, und die anschließende Entfernung der Schutzgruppe(n) umfaßt.

8. Verfahren nach Anspruch 7, dadurch gekennzeichnet, daß die Fluorierung mit einem Addukt

aus Schwefeltrifluorid-sekundärem Amin, vorzugsweise mit Diethylaminoschwefeltrifluorid, durchgeführt wird.

9. Verbindung nach jedem der Ansprüche 1 bis 4 zur Verwendung als Pharmazeutikum, insbesondere als antibakterielles Mittel.

10. Pharmazeutische Zusammensetzung, insbesondere zur Verwendung als antibakterielles Mittel, enthaltend als Wirkstoff eine Verbindung nach jedem der Ansprüche 1 bis 4 zusammen mit einem geeigneten pharmazeutischen Träger.

**Patentansprüche für den Vertragsstaat: AT**

1. Verfahren zur Herstellung von D-(threo)-1-Aryl-2-acylamido-3-fluoro-1-propanol-Verbindungen der allgemeinen Formel (I)

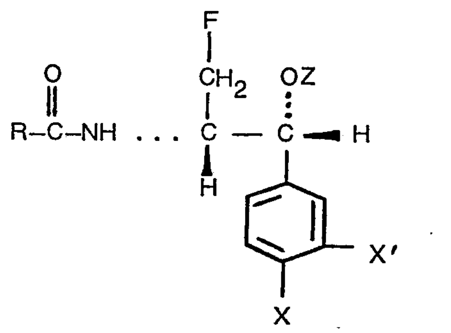

(I),

in der

R für eine Alkyl-Gruppe mit einem oder zwei Kohlenstoff-Atomen, die durch ein oder mehrere Halogen-Atome substituiert sein kann, oder für Azidomethyl, Methylsulfonylmethyl oder eine Halogenodeutero-alkyl-Gruppe steht,

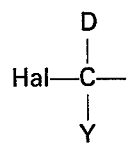

in der Y $-CH_3$, $-CH_2Hal$ oder Hal ist und Hal ein Halogen-Atom bezeichnet, steht,

X und X′ unabhängig voneinander für ein Wasserstoff- oder Halogen-Atom, eine Nitro-, Cyano- oder Phenyl- Gruppe, welch letztere durch Halogen oder durch niederes Alkyl, niederes Alkoxy, Methylsulfonyl oder Nitro substituiert sein kann, oder für eine Gruppe $R_1(A)_n$ stehen, in der n 0 oder 1 ist, A Sauerstoff, Schwefel, Carbonyl, Sulfinyl oder Sulfonyl ist und $R_1$ niederes Alkyl ist oder in dem Fall, in dem A Sulfinyl oder Sulfonyl ist, auch Amino oder niederes Alkylamino sein kann, wobei die vorbezeichneten niederen Alkyl- oder niederen Alkoxy-Gruppen ein bis drei Kohlenstoff-Atome besitzen, und

Z Wasserstoff oder ein Acyl-Rest einer Carbonsäure mit bis zu 16 Kohlenstoff-Atomen ist, sowie der pharmazeutisch unbedenklichen Salze der Verbindungen der Formel (I), in denen Z ein von einer Dicarbonsäure oder Aminosäure mit bis zu 16 Kohlenstoff-Atomen abgeleiteter Acyl-Rest ist, dadurch gekennzeichnet, daß es eines der nachstehenden Verfahren A bis D umfaßt:

A: N-Acylierung eines Amins der allgemeinen Formel (II)

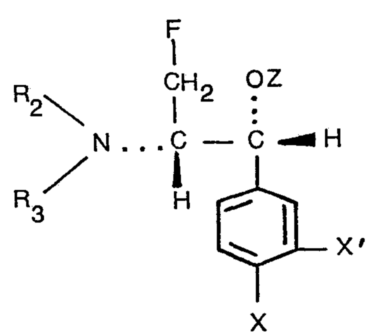

(II),

in der

$R_2$ und $R_3$ jeweils Wasserstoff sind,

X, X′ und Z die für die Formel (I) definierten Bedeutungen haben, wobei besonders bevorzugt Z Wasserstoff ist, mit einer freien Säure oder einem reaktionsfähigen Derivat einer Säure der Formel R—COOH, in der R die für die für die Formel (I) angegebene Bedeutung hat;

B: Ringöffnung, verbunden mit einer Fluorierung in der 3-Stellung der Propan-Struktureinheit, eines Oxazolins der allgemeinen Formel (IV)

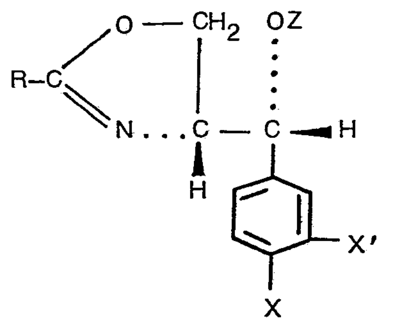

(IV),

in der R, X, X' und Z die für Formel (I) definierten Bedeutungen haben wobei besonders bevorzugt Z Wasserstoff ist;

C: Oxidation eines Ausgangsmaterials, das sich von dem angestrebten Produkt der allgemeinen Formel (I) dadurch unterscheidet, daß es in einer oder beiden der p- und m-Stellungen der Phenyl-Struktureinheit einen Substituenten —$SR_1$ oder —$SOR_1$ trägt, worin $R_1$ die für die Formel (I) definierte Bedeutung hat, so daß eine Umwandlung der bezeichneten Substituenten in —$SOR_1$ bzw. —$SO_2R_1$ bewirkt wird; und

D: Deuterierung eines Ausgangsmaterials der allgemeinen Formel (I), in der R

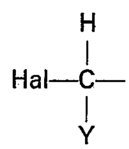

ist, worin Y die für die Formel (I) angegebene Bedeutung hat, unter Bildung der entsprechenden Deuteroverbindung der Formel (I), in der

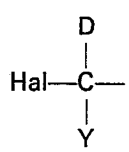

ist, wobei auf das aus den Verfahren A bis D ausgewählte Verfahren, falls erwünscht, eine oder mehrere der Stufen

(i) Veresterung oder Hydrolyse an der 1-Hydroxy-Funktion und

(ii) Bildung eines pharmazeutisch unbedenklichen Salzes, in dem Z ein von einer Dicarbonsäure oder einer Aminosäure mit bis zu 16 Kohlenstoff-Atomen abgeleiteter Acyl-Rest ist.

2. Verfahren nach Anspruch 1, Verfahren A, dadurch gekennzeichnet, daß in dem Amin-Ausgangsmaterial der Formel (II) sowohl Z als auch X' Wasserstoff sind und X Nitro oder die Gruppe $R_1A$ ist, worin A Sulfinyl oder Sulfonyl ist und $R_1$ niederes Alkyl ist oder, wenn A Sulfonyl ist, auch eine freie Amino-Gruppe sein kann.

3. Verfahren nach Anspruch 2, dadurch gekennzeichnet, daß X Nitro oder Methylsulfonyl ist.

4. Verfahren nach Anspruch 1, Verfahren A, Anspruch 2 oder Anspruch 3, dadurch gekennzeichnet, daß in dem reaktionsfähigen Derivat der Säure der Formel R—COOH R für $(Hal)_2CH$— steht, worin die Hal unabhängig voneinander für ein Chlor- oder ein Fluor-Atom stehen.

5. Verfahren nach Anspruch 1, Verfahren A, oder Anspruch 2 bis 4, dadurch gekennzeichnet, daß das reaktionsfähige Derivat der Säure der Formel R—COOH ein niederer Alkylester ist.

6. Verfahren nach Anspruch 1, Verfahren A, oder Anspruch 2 bis 5, dadurch gekennzeichnet, daß das Verfahren weiterhin die Stufe der Herstellung des Ausgangsmaterials der allgemeinen Formel (II) durch Fluorierung in 3-Stellung eines 1,3-Propandiol-Derivats der allgemeinen Formel (III)

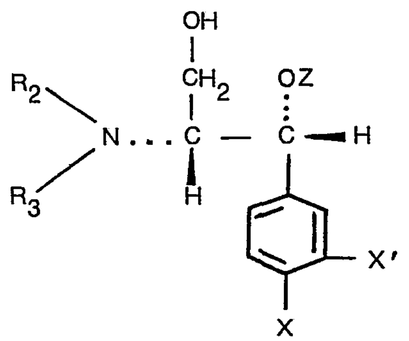

(III),

in der X, X' und Z die in Anspruch 1 definierten Bedeutungen haben, wobei besonders bevorzugt Z Wasserstoff ist, und $R_2$ und $R_3$ einzeln oder gemeinsam Amino-Schutzgruppen bezeichnen, wodurch die 3-Hydroxy-Funktion durch Fluor ersetzt wird, und die anschließende Entfernung der Schutzgruppe(n) umfaßt.

7. Verfahren nach Anspruch 1, Verfahren D, dadurch gekennzeichnet, daß die Deuterierung mittels $CH_3OD$ durchgeführt wird.

8. Verfahren nach irgendeinem der vorhergehenden Ansprüche, dadurch gekennzeichnet, daß eine so erhaltene Verbindung der allgemeinen Formel (I), in der Z Wasserstoff ist, mit einer Aminosäure verestert wird, vorzugsweise mit einer Aminosäure ausgewählt aus Ornithin, Lysin und Glycin, und daß der so erhaltene Ester als solcher oder in Form eines Säureadditionssalzes isoliert wird.

9. Verfahren nach Anspruch 8, dadurch gekennzeichnet, daß der so erhaltene Ester als das Sulfat- oder Hydrochlorid-Salz isoliert wird.

10. Verfahren zur Herstellung einer pharmazeutischen Zusammensetzung, dadurch gekennzeichnet, daß eine Verbindung der allgemeinen Formel (I) oder ein pharmazeutisch unbedenkliches Salz derselben in eine für eine therapeutische Verabreichung geeignete Form gebracht wird.